(19) Europäisches Patentamt
European Patent Office
Office européen des brevets

(11) **EP 1 569 745 B1**

(12) **EUROPÄISCHE PATENTSCHRIFT**

(45) Veröffentlichungstag und Bekanntmachung des Hinweises auf die Patenterteilung:
**20.03.2019   Patentblatt 2019/12**

(51) Int Cl.:
***B01J 8/06*** (2006.01)        ***F28F 9/22*** (2006.01)

(21) Anmeldenummer: **02795180.5**

(22) Anmeldetag: **12.12.2002**

(86) Internationale Anmeldenummer:
**PCT/EP2002/014187**

(87) Internationale Veröffentlichungsnummer:
**WO 2004/052524 (24.06.2004 Gazette 2004/26)**

(54) **MANTELROHRREAKTOR FÜR KATALYTISCHE GASPHASENREAKTIONEN**

SHELL-AND-TUBE TYPE REACTOR FOR CATALYTIC GAS PHASE REACTIONS

REACTEUR TUBULAIRE A ENVELOPPE POUR DES REACTIONS CATALYTIQUES EN PHASE GAZEUSE

(84) Benannte Vertragsstaaten:
**AT BE BG CH CY CZ DE DK EE ES FI FR GB GR IE IT LI LU MC NL PT SE SI SK TR**

(43) Veröffentlichungstag der Anmeldung:
**07.09.2005   Patentblatt 2005/36**

(73) Patentinhaber: **MAN Energy Solutions SE**
**86153 Augsburg (DE)**

(72) Erfinder: **GÜTLHUBER, Friedrich**
**85579 Neubiberg (DE)**

(74) Vertreter: **Paustian, Othmar**
**Boeters & Lieck**
**Patentanwälte**
**Oberanger 32**
**80331 München (DE)**

(56) Entgegenhaltungen:
| | |
|---|---|
| EP-A- 0 382 098 | WO-A-00/54877 |
| DE-A- 1 675 501 | DE-A- 10 024 342 |
| DE-A- 10 137 768 | DE-B- 2 201 528 |
| US-A- 4 357 991 | US-A- 5 161 605 |
| US-A- 5 355 945 | |

**Beschreibung**

Die Erfindung betrifft einen Mantelrohrreaktor gemäß Gattungsbegriff des Patentanspruchs 1 und eine Verwendung gemäß Gattungsbegriff des Patentanspruchs 29. Bevorzugte Ausführungsformen sind gemäß Ansprüche 2-28.

Ein Mantelrohrreaktor ist ein Festbettreaktor, der die Möglichkeit bietet, zwischen dem in dem Festbett reagierenden Prozeßgasgemisch noch innerhalb des Festbetts wie auch zwischen dem Festbett selbst und einem separaten Wärmeträger einen Wärmeaustausch herbeizuführen. Dabei kann die Reaktion prinzipiell sowohl eine endotherme wie auch eine exotherme sein. Das Festbett - im wesentlichen ein granularer Katalysator - befindet sich in den Rohren (Reaktionsrohren) eines allgemein vertikal angeordneten Reaktionsrohrbündels, deren beide Enden abgedichtet in Rohrböden festgelegt sind und die innerhalb eines das Rohrbündel umgebenden Reaktormantels von dem Wärmeträger umspült werden. Das Prozeßgasgemisch wird den Rohren über eine den betreffenden Rohrboden überspannende Reaktorhaube zu- und ebenso über eine den anderen Rohrboden überspannende Reaktorhaube abgeführt. Der Wärmeträger - häufig ein Salzbad - wird mittels einer Umwälzpumpe umgewälzt und von einem Wärmetauscher je nach der Art des Reaktionsprozesses erwärmt bzw. gekühlt. Pumpe wie Wärmetauscher liegen heutzutage gewöhnlich außerhalb des Reaktormantels. Entsprechend tritt der Wärmeträger in der Nähe eines Rohrbodens in den Reaktormantel ein und in der Nähe des anderen Rohrbodens aus ihm aus. Zur Erzielung eines bestimmten, für die Reaktion wünschenswerten Temperaturprofils entlang den Reaktionsrohren können sich Ein- und/oder Austrittsstellen für den Wärmeträger auch noch in dazwischenliegenden Ebenen des Reaktormantels befinden.

Um für sämtliche Rohre des Reaktors - ein moderner Mantelrohrreaktor kann bis zu 30000 Rohre oder mehr enthalten - im Interesse eines einheitlichen Reaktionsablaufs und damit einer hohen Ausbeute und guten Selektivität des Reaktionsprodukts ein möglichst gleiches Temperaturprofil zu erhalten, kommt es darauf an, Temperaturunterschiede im Wärmeträger innerhalb des Reaktormantels klein zu halten und vor allem möglichst gleiche Anströmverhältnisse für alle Rohre zu schaffen. Zu diesem Zweck hat man bereits den Reaktormantel umschließende Ringkanäle mit einer Vielzahl ringsherum verteilter Mantelfenster für die Zu- und Abfuhr des Wärmeträgers sowie im Mantelinneren Verteiler- und Umlenkbleche für den Wärmeträger vorgesehen - vergl. etwa DE-A-2 201 528, wovon im Gattungsbegriff ausgegangen wird -. Während Verteilerbleche eine gewünschte Verteilung des Wärmeträgers über den Reaktorquerschnitt bewirken sollen, dienen miteinander abwechselnde ring- und scheibenförmige Umlenkbleche zur Herbeiführung im wesentlichen quergerichteter Strömungen innerhalb des Rohrbündels, indem sie der global gesehen längsgerichteten Strömung innerhalb des Reaktormantels einen mäanderartigen Verlauf vermitteln.

Mit den betreffenden Ringkanälen und Umlenkblechen sollen, wie gesagt, möglichst gleiche Anströmverhältnisse und damit möglichst gleiche Temperaturprofile für sämtliche Reaktionsrohre des Rohrbündels geschaffen werden. Neben einer hohen Ausbeute und Selektivität bei dem Reaktionsprodukt kann damit bei exothermen Prozessen auch eine Schädigung des Katalysators durch lokale Überhitzung und häufig auch eine Abbrandgefahr vermieden werden, wie sie sich anderweitig in einzelnen Rohren, insbesondere im zentralen Bereich des Reaktors, einstellen und zu großen Schäden führen können. Letzteres spielt eine um so größere Rolle, je mehr das primäre Reaktionsgas im Bestreben, die Ausbeute zu verbessern, mit dem zweiten Reaktanden - zumeist $O_2$ - beladen wird. Besonders kritische Prozesse sind in dieser Hinsicht bekanntermaßen etwa ohne die Herstellung von Acrylsäure, Methacrylsäure, Acrolein, Phthalsäureanhydrid, Maleinsäureanhydrid, Glyoxal, Methylmethacrylat und Acrylnitril sowie die Essigsäurehydrierung.

DE-A-44 31 957 beschreibt in Zusammenhang mit der Herstellung von Acrolein aus Propen eine Prozeßführung, bei der der Temperaturanstieg des Wärmeträgers im Reaktor auf 2 bis 10 °C begrenzt sein soll. Dies soll erreicht werden durch eine Hindurchführung des Wärmeträgers durch den Reaktor global im Gleichstrom mit dem Reaktionsgas, verbunden mit einer verhältnismäßig großen Umwälzmenge. Indessen sind der Umwälzmenge Grenzen gesetzt durch den hohen Pumpenenergiebedarf infolge der insbesondere bei Reaktoren großen Durchmessers und dichter Rohranordnung auftretenden hohen Drosselverluste in der radialen Wärmeträgerströmung wie zum Teil auch durch die Gefahr, daß die Rohre durch zu starke Anströmung seitens des Wärmeträgers in Schwingungen geraten. Ungeachtet dessen ist auch hier noch ein in Betracht fallender Temperaturgradient innerhalb horizontaler Reaktorquerschnitte unvermeidbar.

Ansätze für eine Vergleichmäßigung der Rohranströmung über den Reaktorquerschnitt finden sich bereits in DE-A-1 601 162 und DE-A-1 675 501. Hiernach weisen in der Nähe beider Rohrböden angeordnete, sich über den gesamten Reaktorquerschnitt erstreckende Verteilerbleche im einen Fall einzelne oder auch Gruppen der Reaktionsrohre umgebende Durchtrittsöffnungen, im anderen Fall einzelne Rohre umgebende und/oder zwischen den Rohren liegende Durchtrittsöffnung mit zum Reaktorzentrum hin - entsprechend dem nach dorthin abnehmenden Anströmvordruck - größer werdenden trichterförmigen Ein- und/oder Ausläufen auf. Beide Ausführungen sind nur aufwendig herstellbar, unter Verwendung mehrerer verschiedener Werkzeuge. Auch ist eine verläßliche Bestimmung und Einhaltung des Druckverlustbeiwerts für die jeweilige Geometrie der Durchtrittsöffnungen schwierig. Schließlich aber sind die beiden hier in Rede stehenden Reaktoren sogenannte Längsstromreaktoren, bei denen der Wärmeträger die Reaktionsrohre im wesentlichen längs bestreicht. Die dabei auftretende, im wesentlichen laminare Strömung bewirkt, verglichen mit einer turbulenten Strömung, wie sie bei quergerichteter Rohranströmung entsteht, nur einen recht beschränkten Wärmeübergang. Auch ist auf diese Weise die Erzielung eines gewünschten Temperaturprofils über die Länge der Reaktionsrohre nur sehr beschränkt möglich.

Grundsätzlich unterscheidet man bei Mantelrohrreaktoren Längsstromreaktoren, Querstromreaktoren (bei denen der Wärmeträger einmalig quer durch das Rohrbündel hindurchtritt) und sogenannte Radialstromreaktoren, bei denen der Wärmeträger, wie eingangs ausgeführt, entlang miteinander abwechselnden ring- und scheibenförmigen Umlenkblechen mäanderförmig und damit wiederholt von außen nach innen und von innen nach außen durch das Rohrbündel hindurchtritt. Reine Querstromreaktoren sind nur für kleine Baugrößen denkbar und heute kaum noch gebräuchlich.

Zu den heute üblichen Radialstromreaktoren gibt es zahlreiche Veröffentlichungen mit Auslegungshinweisen und -beispielen, wie etwa VDI-Wärmeatlas, 1997, Abschn. Ob, oder GB-A-310 157. Nach ersterem werden die Rohre entsprechend der Fertigungstoleranz mit engem Spalt, zwischen 0,2 und 0,8 mm, durch die betreffenden Bohrungen in den Umlenkblechen hindurchgeführt. Es werden Konstruktionen mit Teilberohrung bevorzugt, wobei sämtliche Rohre bei ihrer Hindurchführung durch Umlenkbleche abgestützt sind.

Die eingangs angeführte Literaturstelle DE-A-2 201 528 lehrt indessen auch bereits, in den ring- und scheibenförmigen Umlenkblechen um die Rohre herum unterschiedlich bemessene Ringspalte vorzusehen, um durch die dadurch verursachten Leckströme eine möglichst gleichmäßige Strömungsgeschwindigkeit und damit Wärmeübertragung innerhalb der einzelnen Reaktorabschnitte zwischen aufeinanderfolgenden Umlenkblechen zu erreichen. Weitergehende Angaben in diesem Sinn sind EP 0 382 098 B1 zu entnehmen, wonach die Größenänderung der Ringspalte, allerdings in den gesamten Reaktorquerschnitt einnehmenden "Prallplatten" (baffle plates), die insofern eher Verteilerscheiben vergleichbar sind, in Stufen erfolgt.

Durchtrittsöffnungen für den Wärmeträger durch Umlenkbleche, gleichgültig ob es sich dabei um die Reaktionsrohre umgebende Ringspalte, um separate Durchtrittsöffnungen - mit Ausnahme der zentralen Öffnung ringförmiger Umlenkbleche - oder um Mischformen handelt, werden in der nachfolgenden Beschreibung als Teilstromöffnungen bezeichnet.

EP-A-1 080 780 gibt weitere Maßnahmen zur Vergleichmäßigung der Strömungs- und Wärmeverteilung innerhalb horizontaler Reaktorquerschnitte an, wobei im Inneren wie auch an der Peripherie eines ringförmigen Rohrbündels von Umlenkblechen ungestützte Rohre auftreten. Indessen sind solche ungestützten Rohre schwingungsanfällig, womit die Strömungsgeschwindigkeit des Wärmeträgers und entsprechend auch der Wärmeübergang zwischen Rohren und Wärmeträger begrenzt ist. Andernfalls kommt die betreffende Ausführung nur für verhältnismäßig kleine Reaktoren mit vor allem auch geringer Rohrlänge in Betracht.

Im übrigen aber ist die Erzielung gleicher Rohrwandtemperaturen innerhalb einander entsprechender Querschnittsebenen der Rohre, wie sie letztlich für gleiche Reaktionsabläufe entscheidend sind, nicht gleichbedeutend mit der Erreichung gleicher Rohranströmverhältnisse. Vielmehr hat man bei der Passage des Wärmeträgers durch das Rohrbündel hindurch in Rechnung zu stellen, daß der Wärmeträger sich dabei erwärmt bzw. abkühlt. Für die Rohrwandtemperatur gilt

$$T_{Rohrwand} = T_{Wärmeträger} + \frac{q}{\alpha_{Wärmeträger}} \quad (°K),$$

wobei q die prozeßabhängige und damit kaum beeinflußbare lokale Heizflächenbelastung (W/m$^2$) und $\alpha_{Wärmeträger}$ der

vom Wärmeträger und dessen Geschwindigkeit abhängige Wärmeübergangswert $\frac{W}{m2\,°K}$ ist. Um die Rohrwandtemperatur $T_{Rohrwand}$ möglichst weit der Wärmeträgertemperatur $T_{Wärmeträger}$ anzunähern, kommt es darauf an,

$\alpha_{Wärmeträger}$ möglichst groß zu machen. Ist der Term $\frac{q}{\alpha_{Wärmeträger}}$ und somit auch der Unterschied zwischen

$T_{Rohrwand}$ und $T_{Wärmeträger}$ groß, so besteht bei exothermen Reaktionen die Gefahr einer Überhitzung. Dies gilt vor allem für die Stelle des intensivsten Reaktionsablaufs, den sog. Heißpunkt (hot spot).

In erster Näherung gilt

$$\alpha_{Wärmeträger} \sim (W_{quer})^n$$

mit $w_{quer}$ als der Querstromgeschwindigkeit des Wärmeträgers in bezug auf das angeströmte Rohr. Es versteht sich also, daß $w_{quer}$ möglichst groß sein soll. Andererseits sind $w_{quer}$ durch den dafür erforderlichen Leistungsbedarf der Umwälzpumpe Grenzen gesetzt, wie ggf. auch dadurch, daß die Rohre nicht in Schwingungen geraten dürfen.

Mit den erwähnten Teilstromöffnungen in Umlenkblechen läßt sich die Querstromgeschwindigkeit in den angrenzenden Rohrbündelabschnitten beeinflussen. Dabei ist, um durchwegs gleiche Rohrwandtemperaturen in einer jeden horizontalen Ebene zu erreichen, die jeweilige Querstromgeschwindigkeit nicht zwingend konstant.

Von all dem ausgehend liegt der hier in Rede stehenden Erfindung die Aufgabe zugrunde, an allen Reaktionsrohren innerhalb einer jeden horizontalen Querschnittsebene des Reaktors für die Erzielung gleicher Rohrwandtemperaturen optimale Strömungsverhältnisse für den Wärmeträger zu schaffen.

Diese Aufgabe ist erfindungsgemäß maßgeblich mit den Merkmalen des Patentanspruchs 1 gelöst. Die Unteransprüche geben hierzu vorteilhafte Ausgestaltungsmöglichkeiten und Weiterbildungen an, allesamt mit dem Ziel, in sämtlichen Rohren möglichst gleiche, kontrollierbare Temperaturprofile für den Reaktionsablauf herstellen zu können.

Mit den betreffenden Maßnahmen läßt sich innerhalb der einzelnen Querschnittsebenen eine Optimierung der Anströmung der einzelnen Kontaktrohre und damit der darin auftretenden Temperaturprofile erreichen, womit wiederum eine Verbesserung der Ausbeute und Selektivität bzw. andererseits die Möglichkeit erhalten wird, mit einem verhältnismäßig kleinen Reaktor auszukommen. Dazu noch lassen sich die Zeitabstände zwischen fälligen Katalysatorwechseln vergrößern und das Risiko eines "Durchgehens" der Reaktion herabsetzen.

Nachfolgend werden entsprechende Ausführungsbeispiele anhand der Zeichnungen genauer beschrieben. Von diesen zeigt:

Fig. 1 einen etwas schematisierten Längsschnitt eines üblichen Mantelrohrreaktors, wie er für die Verwirklichung der Erfindung geeignet ist,

Fig. 2 einen vergrößerten Ausschnitt aus Fig. 1, wie er dort mit einer gestrichelten Linie umrandet ist,

Fig. 3 einen Ausschnitt aus einem ringförmigen Umlenkblech mit Darstellung von Teilstromöffnungen verschiedener Größe und Dichte in konzentrischen Ringzonen,

Fig. 4a) - f) verschiedene Ausführungen von Teilstromöffnungen in den Umlenkblechen, zum Teil zwischen den Rohrbohrungen und zum Teil an die Rohrbohrungen anschließend,

Fig. 5a) und b) Rohrhindurchführungen durch ein Umlenkblech mit Ringspalten um die Rohre und Abstützungen für die Rohre in den Ringspalten,

Fig. 6a) und b) verschiedene Ausführungen abgedichteter Rohrhindurchführungen,

Fig. 7 einen Teilquerschnitt durch ein ringförmiges Rohrbündel eines Mantelrohrreaktors,

Fig. 8a) und b) Querschnitte eines Mantelrohrreaktors mit unrundem Rohrbündel zum einen mit einem ringförmigen, zum anderen mit einem scheibenförmigen Umlenkblech,

Fig. 9a) und b) je ein vergrößertes Schema der in einem gestrichelt umrandeten Ausschnitt von Fig. 7 erscheinenden Rohre mit Rohrversetzungen ("Rohrtransformationen") in unterschiedlicher Weise.

Fig. 10 ein Rohrmuster mit einer sich stufenweise ändernden Rohrteilung,

Fig. 11 ein Rohrmuster mit einer vereinfachten Rohrtransformation in anderer Weise,

Fig. 12 einen Längsschnitt durch einen Mantelrohrreaktor ähnlich Fig. 1 mit zusätzlichen Organen in Form von eingebauten Gittern,

Fig. 13 eine Strömungsleiteinrichtung am Übertritt zwischen zwei durch Umlenkbleche voneinander getrennten Reaktorabschnitten,

Fig. 14 eine andere Strömungsleiteinrichtung an nämlicher Stelle,

Fig. 15 ein Schema der Strömungsleiteinrichtung aus Fig. 14,

Fig. 16 einen vereinfachten halben Längsschnitt durch einen Mantelrohrreaktor mit zusätzlichen Einbauten bzw. Merkmalen,

Fig. 17 einen vereinfachten halben Reaktorquerschnitt inmitten eines Ringkanals,

Fig. 18a) - c) verschiedene Schaufelformen für einen Ringkanal nach Fig. 17,

Fig. 19 ein Detail aus einem Reaktorlängsschnitt mit quergeschnittenem Ringkanal,

Fig. 20 einen Reaktorlängsschnitt ähnlich Fig. 1 mit hinzugefügten Bypasskanälen für den Wärmeträger,

Fig. 21 einen Reaktorlängsschnitt ähnlich Fig. 1 mit hinzugefügten Entgasungsmitteln für den Wärmeträger und

Fig. 22 einen vereinfachten halben Reaktorlängsschnitt mit angedeuteten unterschiedlichen Katalysatorfüllungshöhen in den einzelnen Reaktionsrohren.

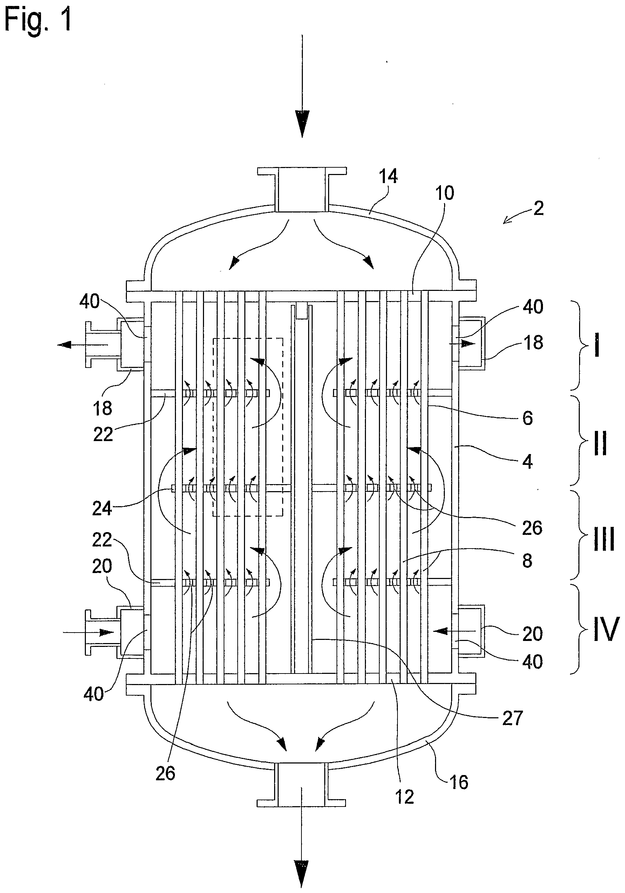

Fig. 1 zeigt, wie gesagt, einen Mantelrohrreaktor, wie er für die Verwirklichung der Erfindung geeignet ist. Der betreffende Mantelrohrreaktor 2 weist ein von einem zylindrischen Reaktormantel 4 umgebenes aufrechtstehendes, ringförmiges Reaktionsrohrbündel 6 auf, dessen Rohre (Reaktionsrohre) 8 an beiden Enden abdichtend in Rohrböden 10 und 12 gehalten sind. Über beide Rohrböden erstrecken sich Reaktorhauben 14 bzw. 16, im dargestellten Beispiel über dem oberen Rohrboden 10 als Gaseintrittshaube und über dem unteren Rohrboden 12 als Gasaustrittshaube für den Einlaß bzw. Auslaß des in den Rohren 8 reagierenden Prozeßgases. Indessen könnte die Strömungsrichtung des Prozeßgases auch umgekehrt sein, in welchem Fall sich die Gasaustrittshaube oben und entsprechend die Gaseintrittshaube unten befinden würde.

Ein jedes der Rohre 8 ist zum wesentlichen Teil mit einem (nicht gezeigten) granularen Katalysator gefüllt und wird von einem gleichfalls nicht gezeigten Wärmeträger - zumeist einer Salzschmelze, zuweilen aber auch Wasser oder einer sonstigen Wärmeträgerflüssigkeit - umspült, der durch eine außerhalb des Reaktormantels 4 angeordnete Umwälzpumpe (nicht gezeigt) durch den Reaktormantel 4 hindurch umgewälzt wird und dabei im Haupt- oder Nebenschluß zu der Umwälzpumpe einen Wärmetauscher (Heizung oder Kühler) durchläuft.

Die Zuführung und Abführung des Wärmeträgers zu dem Reaktormantel 4 bzw. von diesem erfolgt über Ringkanäle 18 und 20 in der Nähe der Rohrböden 10 und 12. Um dem Wärmeträger in bezug auf das Rohrbündel 6 einen im wesentlichen quergerichteten, d.h. radialen Strömungsverlauf zu geben, der in den meisten Fällen schon aus Gründen des besseren Wärmeaustauschs zwischen Wärmeträger und Rohren 8 vorzuziehen ist, befinden sich innerhalb des Reaktormantels 4 in Ebenen zwischen den Ringkanälen 18 und 20 miteinander abwechselnde ring- und scheibenförmige Umlenkbleche 22 bzw. 24, durch die die Rohre 8 zumindest größtenteils hindurchtreten. Indessen weisen die Umlenkbleche 22 und 24 sogenannte Teilstromöffnungen 26 auf, die etwa aus Ringspalten um die Rohre herum oder aber aus separaten Durchbrechungen der Umlenkbleche bestehen können, wovon an späterer Stelle noch ausführlicher die Rede sein wird. Gemäß DE 16 75 501 C3 können die Teilstromöffnungen 26 angefast sein, wobei die Anfasung in Radialrichtung in bezug auf den Reaktor variieren kann.

Wie in Fig. 1 durch die großen Pfeile innerhalb des Reaktormantels 4 symbolisiert, nimmt die Strömung des Wärmeträgers innerhalb des Reaktormantels 4 einen mäanderförmigen Verlauf, wobei sie abwechselnd radial von außen nach innen und von innen nach außen durch das Rohrbündel 6 hindurchtritt. Daneben treten, wie durch die kleinen Pfeile angedeutet, Teilströme des Wärmeträgers über die Teilstromöffnungen 26 durch die Umlenkbleche 22 und 24 hindurch, gewissermaßen den Weg abkürzend. Die Querschnitte der Teilstromöffnungen 26 variieren, radial zu der Reaktormittelachse gesehen, in einer Weise, daß dem mit dem Radius variierenden verfügbaren Durchströmquerschnitt in den einzelnen Reaktorabschnitten I - IV zwischen aufeinanderfolgenden Umlenkblechen 22, 24 Rechnung getragen wird, um so an sämtlichen Rohren 8 optimale Strömungsgeschwindigkeiten für die Erzielung gleicher Temperaturprofile zu erhalten.

Im ganzen gesehen tritt der Wärmeträger in dem gezeigten Beispiel von unten nach oben, das heißt gegensinnig zu dem Reaktionsgas, durch den Reaktor hindurch, doch ist auch eine gleichsinnige Strömungsweise denkbar und zuweilen angebracht.

So weit betrachtet ist der in Fig. 1 dargestellte Reaktor 2 herkömmlicher Art, wie etwa in DE-A-2 201 528 dargestellt. Gleichfalls bekannt (DE-A-25 59 661 und DE-A-27 50 824) ist es, dem Rohrbündel aus strömungstechnischen Gesichtspunkten einen Innendurchmesser von mindestens 20 %, vorzugsweise mindestens 25 % seines Außendurchmessers zu geben, um so bereits unabhängig von Teilstromöffnungen den Unterschied der Radialstromgeschwindigkeiten zwischen Innen- und Außendurchmesser des Rohrbündels zu begrenzen.

Zur Vermeidung von Schwingungen sucht man die Rohre 8 im allgemeinen in den Umlenkblechen abzustützen. Nur bei Reaktoren kleinen Durchmessers, bei geringen Strömungsgeschwindigkeiten des Wärmeträgers oder bei im Verhältnis zu ihrem Durchmesser kurzen Rohren läßt sich auf eine Abstützung der Rohre in zumindest einzelnen der Umlenkbleche verzichten.

Die Umlenkbleche werden in der Regel eine Dicke zwischen 8 mm und 20 mm aufweisen. Während die ringförmigen Umlenkbleche 22 vorzugsweise abgedichtet, etwa zwischen zwei Stützringen (nicht gezeigt), am Reaktormantel angebracht sein werden, können die scheibenförmigen Umlenkscheiben 24, wie gezeigt, zusätzlich von einem zentralen Stützrohr 27 gehalten werden.

Zahl und Abstände der Umlenkbleche sind je nach den Prozeßerfordernissen, wie bereits in DE-A-2 201 528 vorgesehen, variabel. Im allgemeinen werden die Abstände der Umlenkbleche untereinander bzw. gegenüber den Rohrböden oder auch Trennblechen um 30 % bis 300 %, vorzugsweise um 50 % bis 200 % und am zweckmäßigsten um 70 % bis 130 %, bezogen auf ihren mittleren Abstand, variieren.

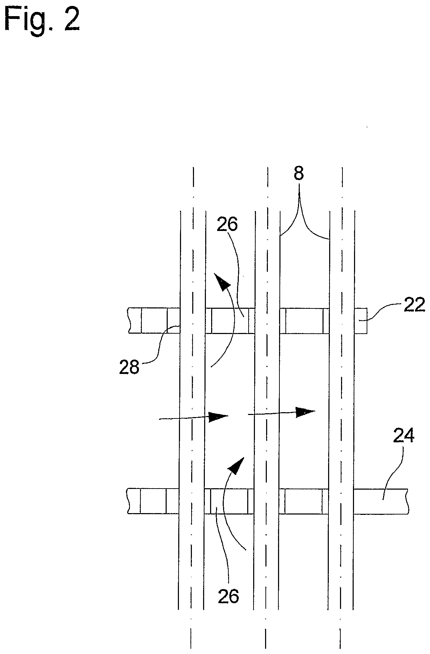

Fig. 2 zeigt ein Detail aus Fig. 1. Die eingezeichneten Pfeile symbolisieren den Strömungsverlauf zwischen aufeinanderfolgenden Umlenkblechen 22 und 24 wie auch durch Teilstromöffnungen 26 hindurch, die hier separat von den Rohrbohrungen 28 dargestellt sind.

Der Durchtrittsquerschnitt aller Teilstromöffnungen 26 auf einem bestimmten Radius nimmt zweckmäßigerweise zur Reaktormitte hin zu, um so die radiale Strömungskomponente des Wärmeträgers zwischen den einzelnen Umlenkblechen 22 und 24 oder, genauer, den Wärmeträgerstrom innerhalb rohrnormaler Ebenen zu optimieren. Dabei können sich die Teilstromöffnungen oder kann sich deren Querschnittszunahme auf eine Art der Umlenkbleche, vorzugsweise die ringförmigen, 22, beschränken.

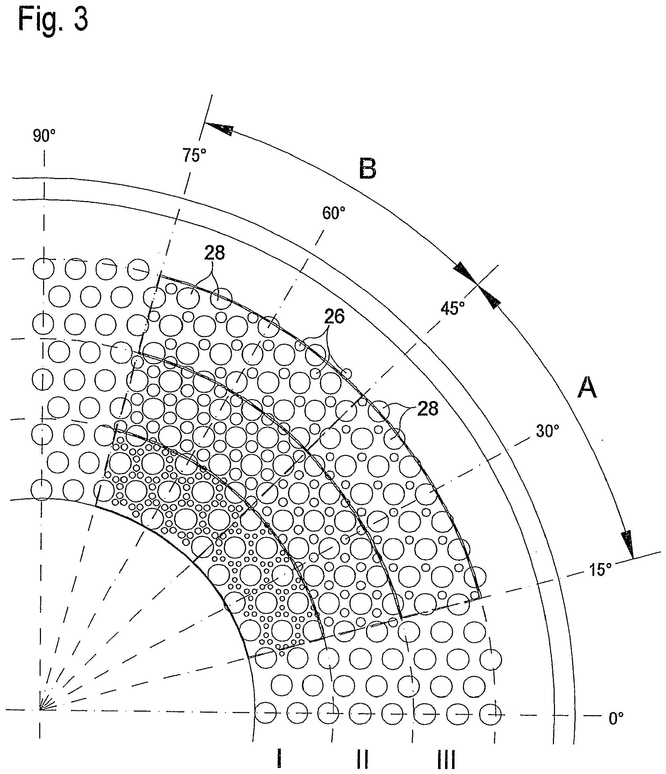

Da es fertigungstechnisch einen unverhältnismäßig großen Aufwand bedeuten würde, die Teilstromöffnungen 26 kontinuierlich mit dem Radius variieren zu lassen, werden sie zweckmäßigerweise, wie in Fig. 3 beispielhaft dargestellt und in ähnlicher Weise in EP-A-0 382 098 angegeben, in Stufen variiert. Wie in Fig. 3 angedeutet, können die Teilstromöffnungen dabei nach Größe und Anzahl pro Flächeneinheit variieren. Ihre Anzahl pro Flächeneinheit kann zumindest bereichsweise kleiner oder größer sein als diejenige der Rohrbohrungen 28. Dabei können die betreffenden Teilstromöffnungen sich in irgendeiner mehr oder weniger regelmäßigen Verteilung zwischen den Rohrbohrungen befinden, oder aber es können einzelne Teilstromöffnungen mehrere Rohre umgeben.

Gemäß Fig. 3 sind die radialen Abmessungen der dort gezeigten Zonen I, II und III ebenso wie die Öffnungswinkel der Sektoren A und B jeweils gleich. Indessen können beide ebenso wie die Zahl der Zonen bzw. unterschiedlichen Sektoren variieren. Zweckmäßigerweise erfolgt der Übergang zu einer anderen Zone bzw. einem anderen Sektor, wo sich der rechnerische Durchtrittsquerschnitt der Teilstromöffnungen um mehr als 30 % verändert hat.

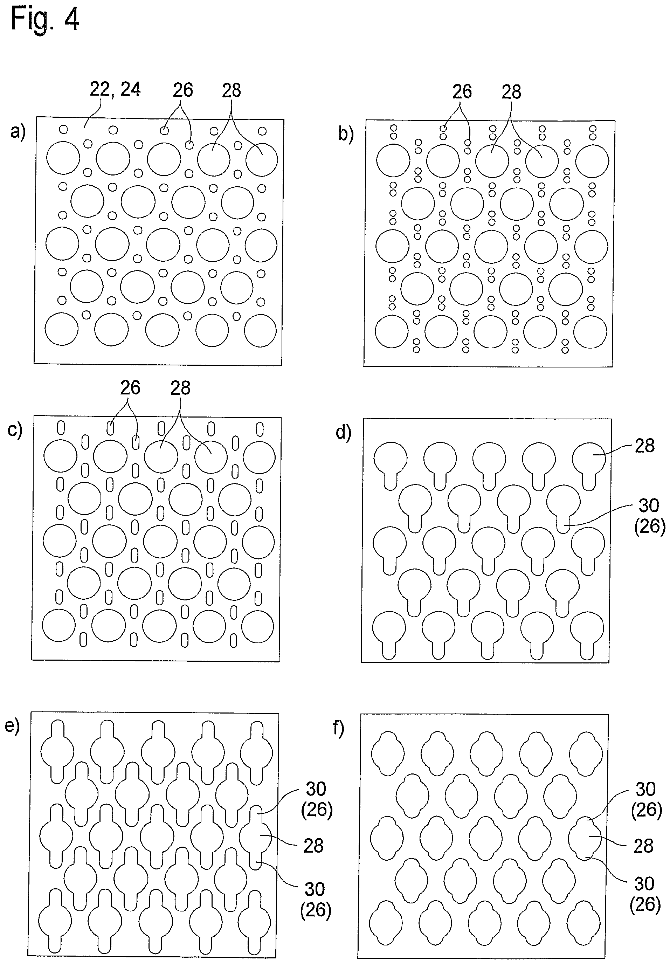

Da sich die Teilstromöffnungen 26 nach Fig. 3 außerhalb der Rohrbohrungen 28 befinden, sind die Rohre ungeachtet der Teilstromöffnungen in den Umlenkblechen abgestützt. Prinzipiell können die Teilstromöffnungen 26, wie gesagt, separat von den Rohrbohrungen 28 vorgesehen sein, wie dies etwa in Fig. 4a) - c) gezeigt ist, wobei sie auch eine andere als eine kreisrunde Form aufweisen können (Fig. 4c)). Andererseits können die Teilstromöffnungen 26 aber auch mit den Rohrbohrungen 28 vereint sein, d.h. sich an diese anschließen (Figuren 4d) - f)). Dazu können die Rohrbohrungen 28 ein- oder mehrseitig Erweiterungen 30 aufweisen, deren Breite zweckmäßigerweise geringer ist als der Durchmesser der Rohrbohrungen. So oder so können die Teilstromöffnungen 26 nichtsdestoweniger in ihrer Größe variieren (Fig. 4a) - f)). Dabei beschränkt sich ihre Form keineswegs auf die gezeigten Beispiele.

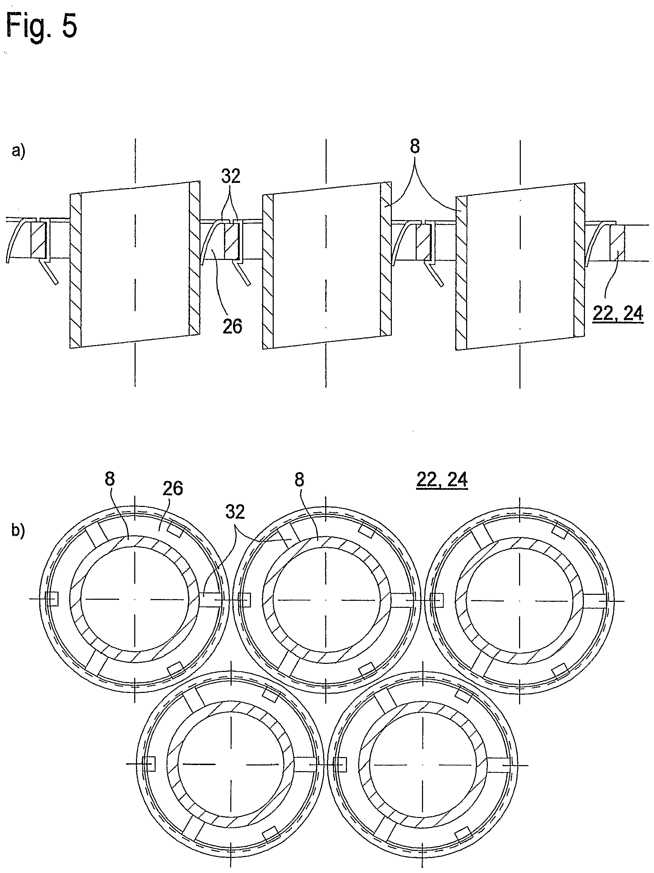

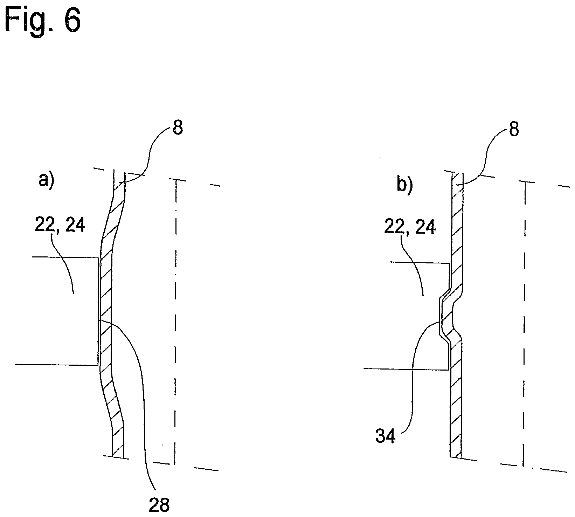

Sofern die Teilstromöffnungen 26 gemäß Fig. 5 aus Ringspalten um die Rohre 8 herum bestehen, werden die Rohre darin zweckmäßigerweise durch geeignete Abstandshalter 32 abgestützt. Werden die Rohre 8 jedoch durch dazu passende Rohrbohrungen 28 hindurchgeführt, so sollte dies aus Abstützungsgründen mit möglichst geringem Spiel erfolgen. Sollen die Spalte aus Durchsatzbemessungsgründen geringer sein als dies fertigungstechnisch mit vertretbarem Aufwand erreichbar ist, so können die Rohren in den betreffenden Rohrbohrungen 28 gemäß Fig. 6a) aufgeweitet werden, wie auch dies wiederum, allerdings für die Rohrhindurchführung durch Trennbleche, aus DE-A-2 201 528 bekannt ist. Dabei kann die Aufweitung auch zur noch vollkommeneren Abdichtung gemäß Fig. 6b) in eine Ringnut 34 der Rohrbohrung hinein erfolgen.

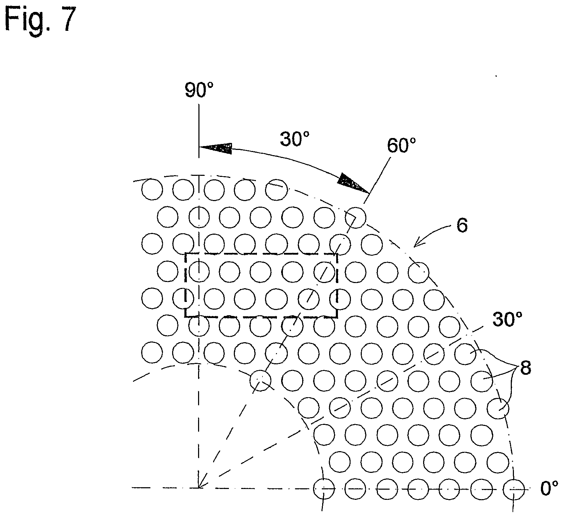

Nun tritt aber bei Mantelrohrreaktoren nicht nur das Problem einer radial ungleichmäßigen Strömungsgeschwindigkeit auf. Wie am besten aus Fig. 7 ersichtlich, werden die Reaktionsrohre 8 üblicherweise mit einer Teilung entsprechend gleichseitigen Dreiecken angeordnet. Wird ein solches Rohrbündel 6 radial von innen nach außen oder umgekehrt durchströmt, so findet der Wärmeträger darin unterschiedliche Strömungswiderstände vor, je nachdem, in welcher Achsrichtung er fließt. Richtungen (radiale Achsen), in welchen die Rohre 8 miteinander fluchten (nach Fig. 7 etwa bei 0°, 60° usw.), wechseln mit solchen ab, in denen die Rohre mehr oder weniger gegeneinander versetzt auftreten. Während der Wärmeträger bei miteinander fluchtenden Rohren 8 eine verhältnismäßig geringe Ablenkung erfährt, wird er bei gegeneinander versetzten Rohren stärker umgelenkt, wobei sich ein höherer Druckverlust ergibt, der die Strömungsgeschwindigkeit herabsetzt. Diese Verhältnisse wiederholen sich in Winkelabständen von 60°, bezogen auf die Mittelachse des Rohrbündels. Die ungleichen Anströmverhältnisse an den Rohren 8 führen zu einem ungleichmäßigen Wärmeübergang und dementsprechend ungleichmäßigen Rohrtemperaturprofil über den Umfang des Rohrbündels 6.

Zur Erreichung eines über den Umfang im wesentlichen homogenen Rohrtemperaturfeldes bestehen verschiedene Möglichkeiten: Entweder wird die Größe oder Dichte der Teilstromöffnungen in Umfangsrichtung den unterschiedlichen Anströmverhältnissen angepaßt, oder es wird die radiale Stärke des Rohrbündels 6 über dessen Umfang variiert, oder es werden die Rohrabstände variiert. All diese Maßnahmen können auch miteinander kombiniert werden.

In Fig. 3 sind in Radialrichtung wie auch in Umfangsrichtung des Rohrbündels 6 variierende Teilstromöffnungen 26 zu erkennen. Das heißt, es treten in miteinander abwechselnden Sektoren A und B von jeweils 30° unterschiedlich große und/oder dicht angeordnete Teilstromöffnungen 26 auf, wobei sich deren Anordnung an den Mittelachsen der Sektoren spiegelt. Ebenso wie für die Variation der Teilstromöffnungen 26 in radialer Richtung gilt auch für diejenige in Umfangsrichtung, daß aus fertigungstechnischen Gründen eine stufenweise Variation einer kontinuierlichen vorzuziehen ist. Prinzipiell können auch mehr als zwei unterschiedliche Sektoren wie die Sektoren A und B auftreten. Auch kann sich ihre Breite unterscheiden, doch sind zweierlei gleich breite Sektoren von jeweils 30° vorzuziehen.

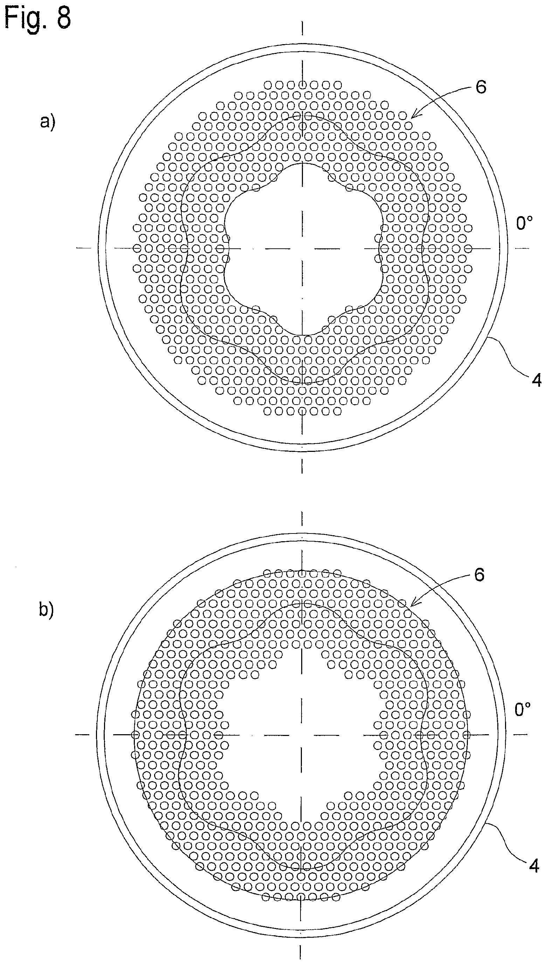

Die zweite Möglichkeit zur Vergleichmäßigung der Rohranströmung entlang radialen Achsen ist in Fig. 8 veranschaulicht. Hiernach variiert die radiale Stärke des Rohrbündels 6 entlang dem Umfang desselben. Prinzipiell können innere und/oder äußere Kontur des Rohrbündels variieren. Die Figuren 8a und 8b zeigen beispielhaft die Positionen dazu passender ring- bzw. scheibenförmiger Umlenkbleche.

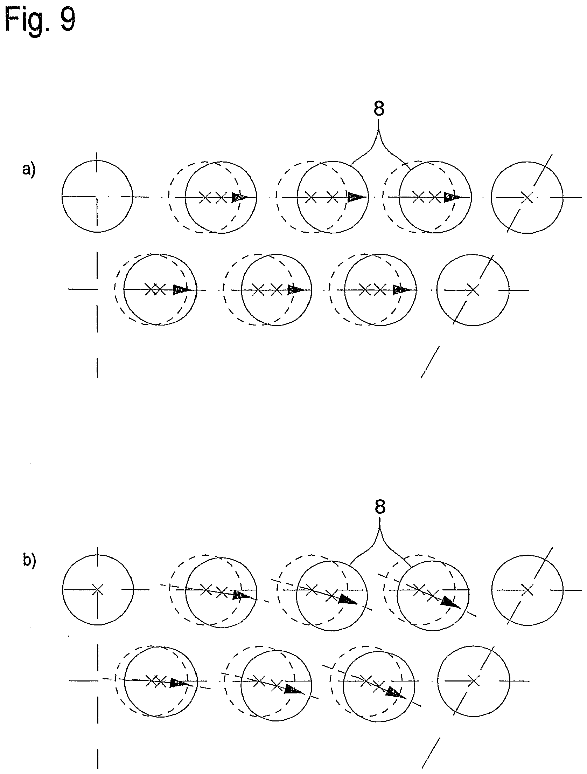

Als dritte Möglichkeit kann die Rohrteilung über den Umfang variieren. In Fig. 7 ist schematisch beispielhaft ein Ausschnitt eines Rohrbündels 6 mit regelmäßiger dreieckförmiger Rohrteilung gezeigt. Dabei treten, wie gesagt, geradlinige Rohrzeilen mit miteinander fluchtenden Rohren beispielsweise entlang den 0°- und 60°-Achsen auf, während entlang den 30°- und 90°-Achsen gegeneinander versetzte Rohre zu erkennen sind, an denen der Wärmeträger einen größeren Widerstand vorfindet. Um dem zu begegnen, sieht Fig. 9 eine teilweise Versetzung der Rohre 8 gegenüber dem regelmäßigen Rohrmuster nach Fig. 7 vor. Die Figuren 9a) und b) zeigen jeweils einen Ausschnitt, wie er in Fig. 7 gestrichelt eingegrenzt ist. Dabei läßt Fig. 9a) eine Versetzung der Rohre ("Rohrtransformation") entlang zur 0°-Achse parallelen geradlinigen Rohrzeilen für Rohre zwischen den 60°- und 90°-Achsen erkennen. Diese Rohrtransformation erfolgt selbstredend entlang sämtlichen tangentialen Rohrzeilen eines jeden 60°-Rohrbündelsektors.

Nach Fig. 9b) sind die Rohre 8 anstatt entlang geradlinigen Rohrzeilen entlang Umfangslinien versetzt. Hier wie dort sind naturgemäß die Rohre auf den 0°-, 30°-, 60°-Achsen usw. nicht betroffen.

Fig. 7 zeigt, wie gesagt, nur ein Schema, während die Zahl der Rohre und entsprechend deren Dichte in praktischen Fällen wesentlich größer sein wird. Heutzutage üblich sind, wie erwähnt, schon Rohrzahlen von beispielsweise 30000 für einen Reaktor. Entsprechend ist es üblich, in den Rohrböden wie auch Umlenkblechen jeweils mehrere, beispielsweise 10 Rohre innerhalb einer Rohrzeile gleichzeitig zu bohren.

In dieser Hinsicht stellt die vorausgehend beschriebene Rohrtransformation zwar einen Mehraufwand dar, doch kann auch sie bei großer Rohrzahl stufenweise erfolgen. Die jeweilige Rohrversetzung selbst bereitet unter Verwendung neuzeitlicher, computergesteuerter Bohrgeräte keine Schwierigkeiten. Dabei ist die Rohrtransformation entlang von Querlinien, d.h. in Richtung der betroffenen Rohrzeilen, gemäß Fig. 9a) vorzuziehen. In jedem Fall verbessert die beschriebene Rohrtransformation die Gleichförmigkeit der Rohranströmung und damit auch der Temperaturen innerhalb der Rohre in rohrnormalen Ebenen, womit sich wiederum Ausbeute und Selektivität des Reaktionsprodukts verbessern bzw. mit einem kleineren Reaktor auskommen läßt.

In der Praxis wird bei der Rohrtransformation so verfahren, daß zunächst, ausgehend von einem Nenn-Teilungsabstand unter Berücksichtigung eines minimalen zulässigen Teilungsverhältnisses eine minimale und eine maximale Rohrteilung festgelegt werden. Auf der Achse mit versetzter Rohranordnung ist die größere Rohrteilung, auf der Achse mit fluchtender Rohranordnung die minimale Rohrteilung vorzusehen. Ausgehend von den genannten Randbedingungen kann sich die Teilung nun stetig oder auch stufenweise ändern. Dabei wird die maximale Rohrteilung zweckmäßigerweise auf einen Wert $< 1,3 \, (T_{nenn} - D_a) + D_a$ festgelegt mit $T_{nenn}$ als der Nennteilung und $D_a$ als dem Außendurchmesser der Rohre. Analog dazu wird die minimale Teilung ermittelt. Wie ersichtlich tritt die maximale Teilung beispielsweise bei den 30°- und 90°-Achsen, die minimale Teilung bei den 0°- und 60°-Achsen auf. Dazwischen kann sich die Teilung gleichmäßig ändern.

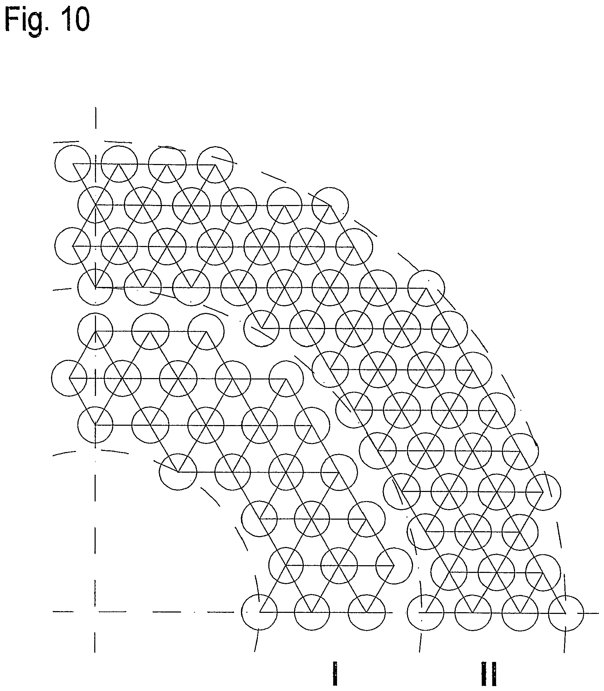

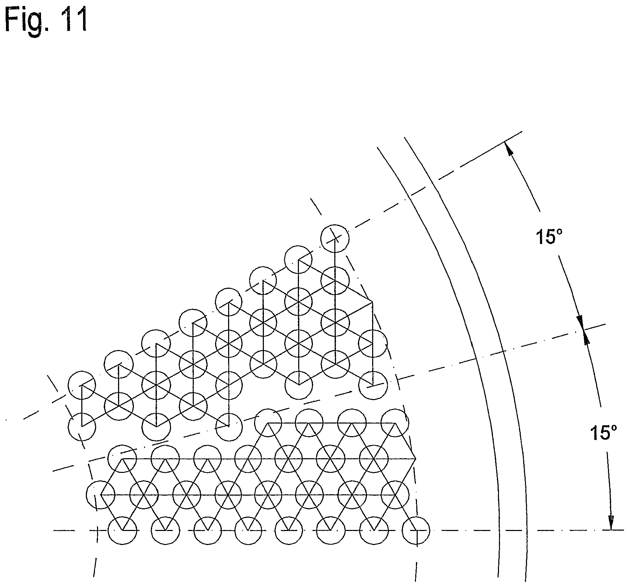

Wo keine computergesteuerten Bohrgeräte zur Verfügung stehen, kann eine Art Rohrtransformation auch dadurch erfolgen, daß Zonen mit regelmäßigen, jedoch verschieden dichten Rohrmustern gemäß Fig. 10 radial und/oder gemäß Fig. 11 in Umfangsrichtung aneinanderschließen. Dabei können sich ergebende Zwischenräume, nach Fig. 11 etwa entlang der 15°-Achse, willkürlich mit Rohren ausgefüllt werden.

Eine Rohranordnung mit radial aneinanderschließenden Zonen unterschiedlicher Berohrung ist an sich bereits aus US-A-4,357,991 bekannt. Hiernach allerdings sind die Rohre auf konzentrischen Rohrkreisen mit jeweils gleichen tangentialen Rohrabständen angeordnet, wobei sich die Anzahl der Rohre pro Rohrkreis stufenweise zwischen jeweils mehrere Rohrkreise umfassenden Zonen ändert.

Neben den vorgenannten Maßnahmen können in der Berohrung auch nach DE 199 09 340 A1 Zonen mit unterschiedlichen Rohrdurchmessern Verwendung finden, wobei der Rohrinnendurchmesser etwa von 25 mm im radial innenliegenden Bereich bis 30 mm im äußeren Bereich variieren kann. Zusätzlich werden im Rohrbündel 6 sogenannte "Thermorohre" mit Temperatur- und ggf. sonstigen Meßstellen auftreten, die den gleichen oder auch einen anderen Durchmesser haben können wie die benachbarten Reaktionsrohre.

Wie weiter oben ausgeführt, bildet sich bei radialer, d.h. rohrnormaler Anströmung der Rohre 8 durch den Wärmeträger eine turbulente Strömung aus. Dies gilt jedoch nicht ohne weiteres bereits für die zuerst, etwa beim Eintritt des Wärmeträgers aus einem Ringkanal oder beim Übertritt des Wärmeträgers von einem Reaktorabschnitt zu dem nächsten angeströmten Rohr. Hier ist die Strömung zunächst noch mehr oder weniger laminar - ein weiterer Grund für einen normalerweise ungleichförmigen Wärmeübergang in rohrnormalen Ebenen -.

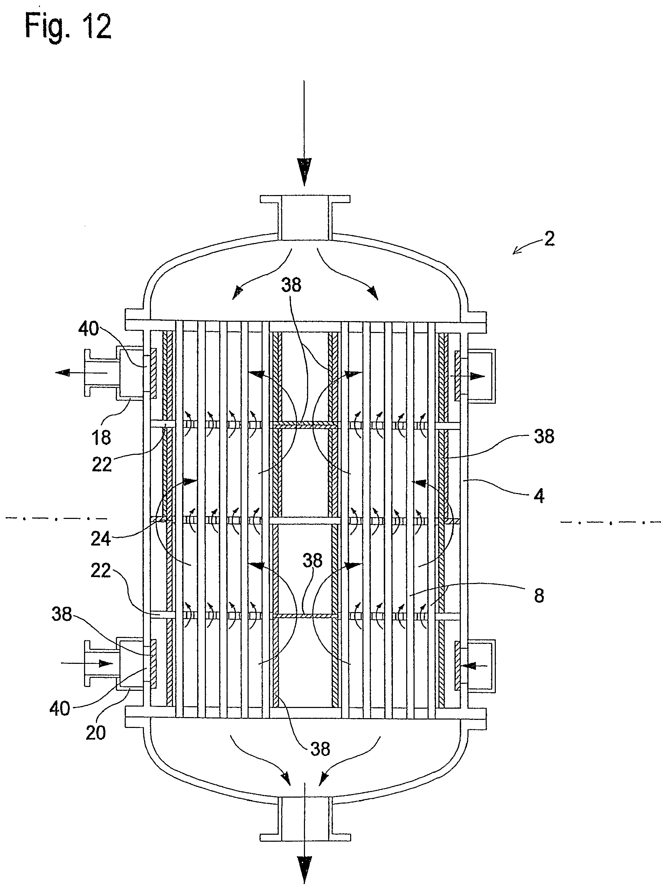

Um dem zu begegnen sieht Fig. 12 bei einem Mantelrohrreaktor ähnlich dem in Fig. 1 gezeigten vor, an allen insoweit kritischen Stellen Gitter 38 in Form von turbulenzerzeugenden Gittern ("Turbulenzgitter") aber auch als Strömungsverteilungsgitter anzubringen. Beide Funktionen können in einem einzigen Gitter vereinigt sein. Ebenso aber kann das jeweilige Gitter 38 auch, wie in der oberen Hälfte von Fig. 12 gezeigt, zwei-(oder auch mehr-)schichtig sein.

Entsprechende Gitter 38 können auch, wie gezeigt, an den Fenstern 40 des wärmeträgereintrittsseitigen Ringkanals 20 oder, horizontal verlaufend, radial innerhalb und/oder außerhalb der Umlenkbleche 22, 24 angeordnet sein.

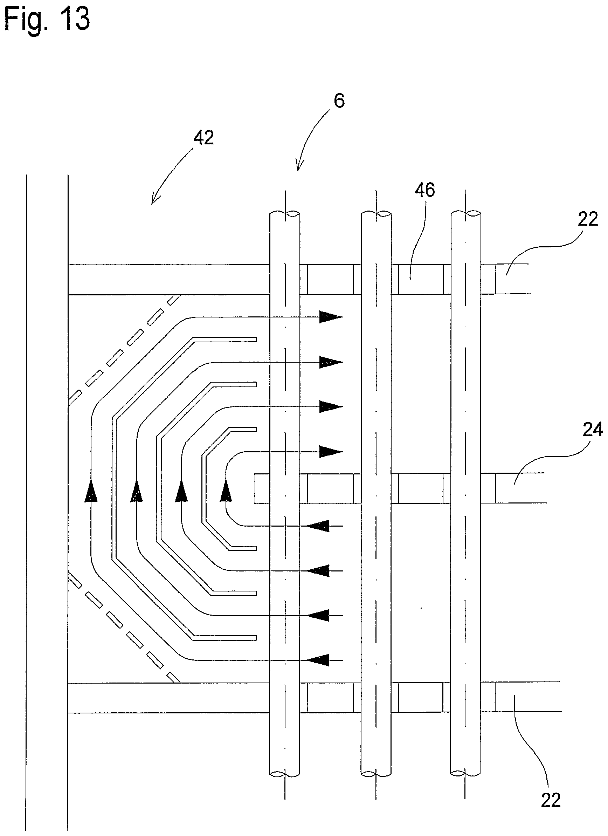

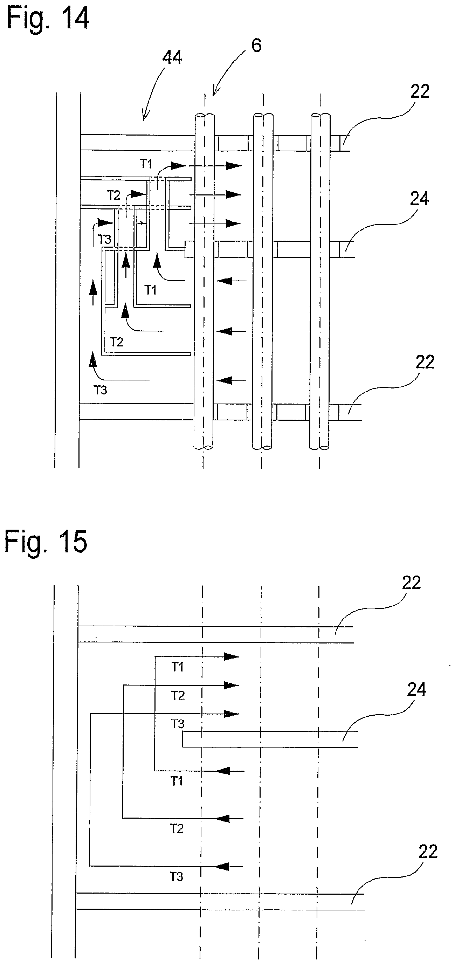

Um des weiteren eine in gewünschter Weise geschichtete Strömung zwischen den Umlenkblechen 22 und 24 wie auch den Umlenkblechen und benachbarten Rohrböden zu erhalten, können am Übergang zwischen den davon begrenzten horizontalen Reaktorabschnitten - z.B. I, II, III und IV in Fig. 1 - Strömungsleiteinrichtungen vorgesehen sein, wie sie in den Figuren 13 und 14 dargestellt sind. Während die Strömungsleiteinrichtung 42 nach Fig. 13 die Aufeinanderfolge übereinanderliegender Strömungsschichten umkehrt, bewirkt die Strömungsleiteinrichtung 44 nach Fig. 14, wie sie in Fig. 15 schematisiert dargestellt ist, eine Beibehaltung der Aufeinanderfolge der Strömungsschichten etwa in dem Sinn, daß die unterste Strömungsschicht T1 die unterste Strömungsschicht bleibt, usw.. Strömungsleiteinrichtungen mit analoger Strömungsführung können ebenso im Reaktorzentrum wie an der Peripherie angeordnet sein. Ferner können an die Stelle solcher Strömungsleiteinrichtungen im Bedarfsfall auch Mischer treten. Die betreffenden Mischer können mit Turbulenz- und/oder Strömungsverteilungsgittern kombiniert sein und dergl. mehr. Wo das Rohrbündel 6, wie etwa in Fig. 8 gezeigt, unrund ist, können die beschriebenen Einbauten wie Gitter 38, Strömungsleitbleche 48, Strömungsleiteinrichtungen 42 bzw. 44, Mischer oder dergl. der Kontur des Rohrbündels folgen.

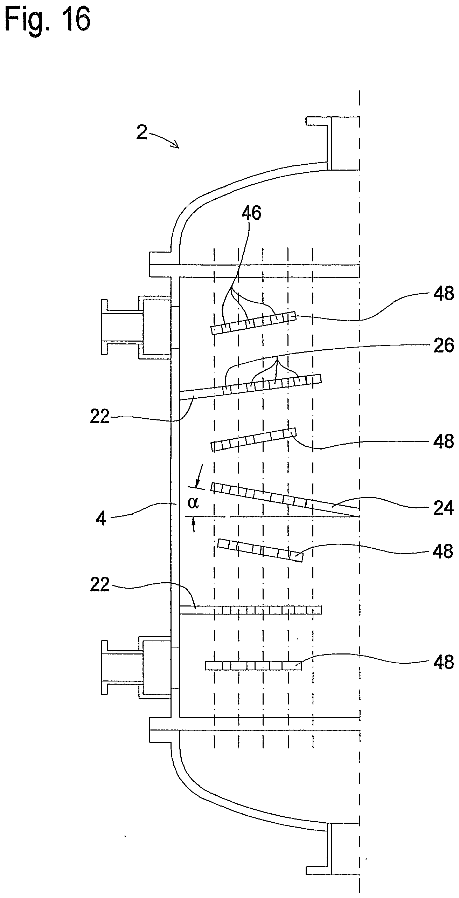

Fig. 16 sieht zur weiteren Beeinflussung der Wärmeträgerströmung im Bereich des Rohrbündels 6 zwischen den Umlenkblechen 22 und 24 wie auch zwischen Umlenkblechen 22 und benachbarten Rohrböden 10 bzw. 12 zusätzliche, gewünschtenfalls mit Teilstromöffnungen 46 ähnlich den Teilstromöffnungen 26 versehene, ggf. konisch geneigte Strömungsleitbleche 48 vor. Wie diese können - in Fig. 16 gleichfalls gezeigt - auch einzelne oder alle der Umlenkbleche 22, 24 konisch geneigt sein, wobei der Konuswinkel $\alpha$ zweckmäßigerweise kleiner als 30°, vorzugsweise kleiner als 20° und am zweckmäßigsten kleiner als 15° ist. Wie diejenigen von Umlenkblechen 22, 24, Einbauten wie Gittern 38, Strömungsleiteinrichtungen 42, 44 oder Mischern 42, 44 und dergl. können auch die Konturen der Strömungsleitbleche 48 ggf. den Konturen eines unrunden Rohrbündels 6 nach Fig. 7 folgen. Im übrigen aber werden die Abmessungen der Strömungsleitbleche 48 in Radialrichtung in der Regel geringer sein als diejenigen der ringförmigen Umlenkbleche 22.

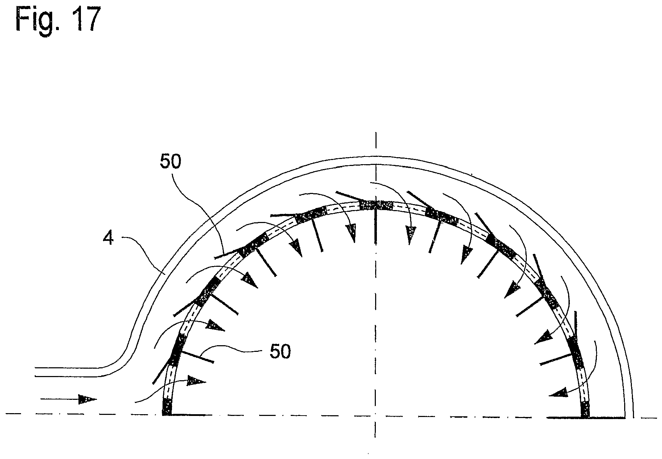

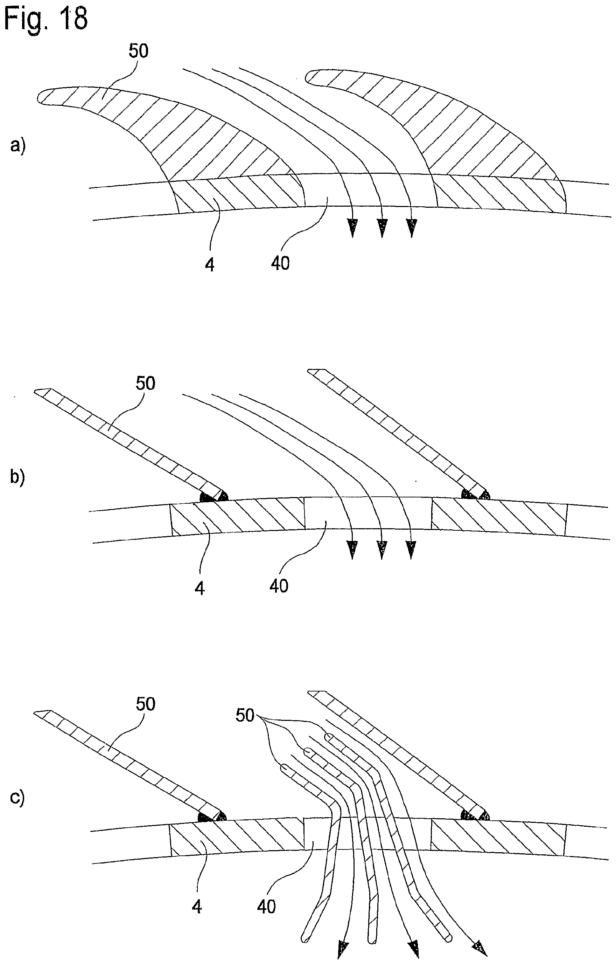

Die Figuren 17 und 18 zeigen Möglichkeiten, die Fenster 40 des eingangsseitigen Ringkanals, z.B. 20, mit Schaufeln 50 zur Herbeiführung einer gewünschten Verteilung wie auch Ausrichtung der auf das Rohrbündel 6 auftreffenden Wärmeträgerströmung auszurüsten. Derartige Schaufeln vermögen zudem, den Strömungswiderstand beim Hindurchtritt durch die betreffenden Fenster und damit auch die seitens der Umwälzpumpe aufzubringende Pumpenleistung herabzusetzen. Die Figuren 18a) - c) zeigen Schaufeln 50 beispielhaft in verschiedenen Ausführungen, als profilierte oder aber aus Blech geformte Schaufeln.

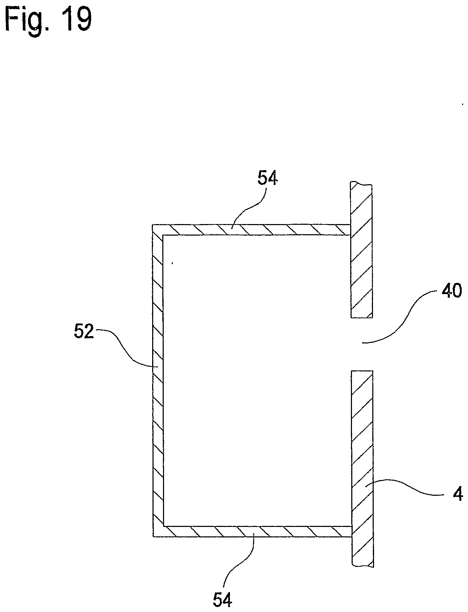

Eine noch weitere Möglichkeit, die Radialströmung in der Ebene eines Ringkanals, vor allem des wärmeträgereintrittsseitigen Ringkanals, in gewünschter Weise zu steuern, besteht darin, Größe, Form oder Abstand der betreffenden Fenster 40 zu variieren. Dabei kann gemäß Fig. 19 ein einziges durchgehendes, in seiner Höhe variables Fenster 40 an die Stelle der herkömmlichen Vielzahl von Fenstern treten, wobei dieses dann zugleich eine Dehnfuge für den Reaktormantel 4 bilden kann. Die unvermeidlichen Wärmedehnungen bzw. -kontraktionen des Reaktormantels werden in diesem Fall von den - gewöhnlich dünneren und entsprechend flexiblen - Wänden 52 und 54 des Ringkanals aufgenommen. Auch ein solch durchgehendes Fenster kann selbstredend noch mit einem Gitter oder mit Schaufeln ausgestattet werden, wie vorausgehend beschrieben.

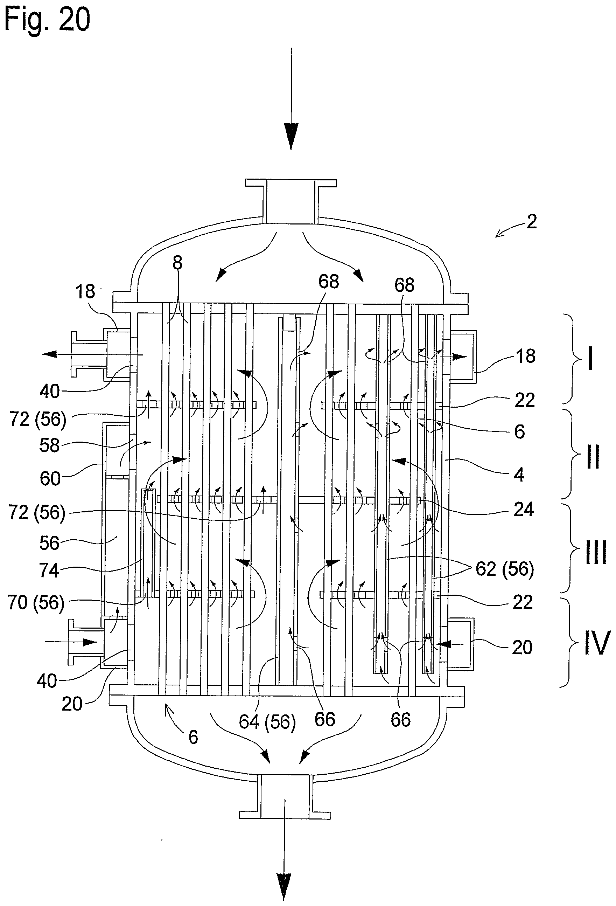

Exotherme, vor allem oxidative katalytische Gasphasenreaktionen neigen dazu, zeitweise sehr heftig abzulaufen, was zur Ausbildung eines "Heißpunkts" (Hot Spot), zumeist in der Nähe des gaseintrittsseitigen Rohrbodens, führt. Im Bestreben, die Wirksamkeit der vorausgehend beschriebenen Maßnahmen nicht durch Nebeneffekte zu verringern, wie es beim Entstehen eines ausgeprägten Heißpunkts der Fall wäre, können Kurzschlußströme vorgesehen werden, die noch nicht oder wenig aufgeheizten Wärmeträger gezielt zu Stellen des Heißpunkts führen. Zu diesem Zweck sind gemäß Fig. 20 Bypasskanäle 56 in mehreren Ausführungen vorgesehen. Beispielsweise (linke Hälfte der Darstellung) können solche Bypasskanäle 56 sich außenseitig an dem Reaktormantel 4 befinden, etwa, wie gezeigt, anschließend an den wärmeträgereintrittsseitigen Ringkanal 20, und durch Fenster 58 ähnlich den Fenstern 40 der Ringkanäle 18 und 20 hindurch ins Innere des Reaktormantels 4 münden - im gezeigten Fall im Reaktorabschnitt II -. Gewünschtenfalls können solche Bypaßkanäle, wie in Fig. 20 dargestellt, über einen eigenen Ringkanal 60 führen. So oder so können auch die Fenster 58 gewünschtenfalls wie die Fenster 40 Gitter ähnlich den Gittern 38 oder Schaufeln ähnlich den Schaufeln 50 aufweisen. Des weiteren können Bypasskanäle 56 gemäß Fig. 20, rechte Hälfte, auch durch eigene Bypassrohre 62 im Inneren des Reaktormantels 4 wie auch ein - sehr häufig vorgesehenes - Zentralrohr 64 verwirklicht sein, woran Ein- und Austrittsöffnungen 66 bzw. 68 für den hindurchgeleiteten Wärmeträger an ganz beliebigen Stellen auftreten können. WO 90/06807 offenbart zu einem ähnlichen Zweck bereits eigene zentrale Bypassrohre.

Anzustreben ist grundsätzlich, die betreffenden Kurzschlußströme möglichst gleichmäßig über den Reaktorumfang zu verteilen. Dazu können Bypasskanäle 56 auch in den Umlenkblechen 22, 24 von entsprechenden Aussparungen 70 oder 72 radial außerhalb bzw. innerhalb des Rohrbündels 6 gebildet werden. Dabei können, wie gezeigt, an die betreffenden Aussparungen Bypassrohre 74 anschließen, die sich gewünschtenfalls ähnlich den Rohren 62 und 64 über ein oder mehrere benachbarte Umlenkbleche hinweg erstrecken und ggf. Ein- oder Austrittsöffnungen an beliebiger Stelle aufweisen. Andererseits besitzen insbesondere einfache Aussparungen eine häufig wünschenswerte Mischfunktion. In DE 100 24 342 A1 und über den Reaktorumfang verteilte Bypassöffnungen innerhalb eines durch eine horizontale Trennwand in zwei übereinanderliegende Abschnitt unterteilten wärmeträgereintrittsseitigen Ringkanals offenbart, die durch Umlenkbleche voneinander getrennte Reaktorabschnitte speisen. Hier wie dort kann der Durchtrittsquerschnitt der Bypassöffnungen bzw. -kanäle veränderbar sein, so z.B. um einer Heißpunktverlagerung infolge Katalysatoralterung Rechnung zu tragen.

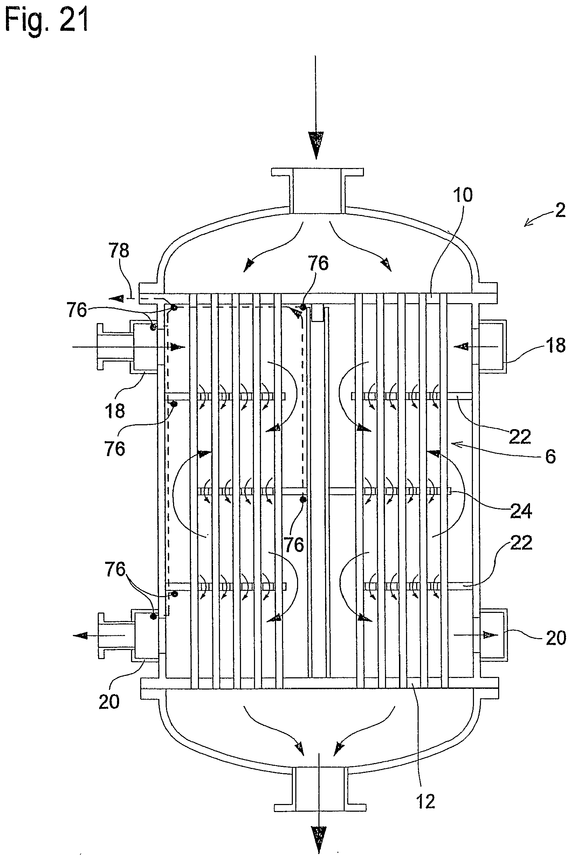

Fig. 21 zeigt schließlich noch - wiederum im Sinne der Herbeiführung kalkulierbarer Strömungen innerhalb des Reaktors 2 - die Anordnungen von Entgasungsorganen 76 an allen möglichen hierfür denkbaren Stellen innerhalb des Reaktors, so etwa unterhalb der Umlenkbleche 22 und 24 und des obenliegenden Rohrbodens 10 - was insbesondere bei von oben nach unten gerichteter Strömung des Wärmeträgers von Bedeutung ist - sowie in den Ringkanälen 18, 20 und ggf. 56 (Fig. 20). Die betreffenden Entgasungsorgane 76 können, erforderlichenfalls über Drosselorgane (nicht gezeigt), mit einer gemeinsamen Gasabführungsleitung 78 verbunden sein, die einen Gas-Flüssigkeits-Abscheider enthalten kann und beheizt sein sollte. Die Gasabführungsleitung 78 kann in das Umwälzpumpengehäuse oder ein separates Entgasungsgefäß führen. Genaueres über derartige Maßnahmen läßt sich den Veröffentlichungen DIN 4754 und VDI-Richtlinie 3033 entnehmen. Indessen kann der Wärmeträger in Gestalt eines Salzbades zumindest außerhalb des Reaktors bewußt mit einem Inertgas wie z.B. Stickstoff überlagerbar sein.

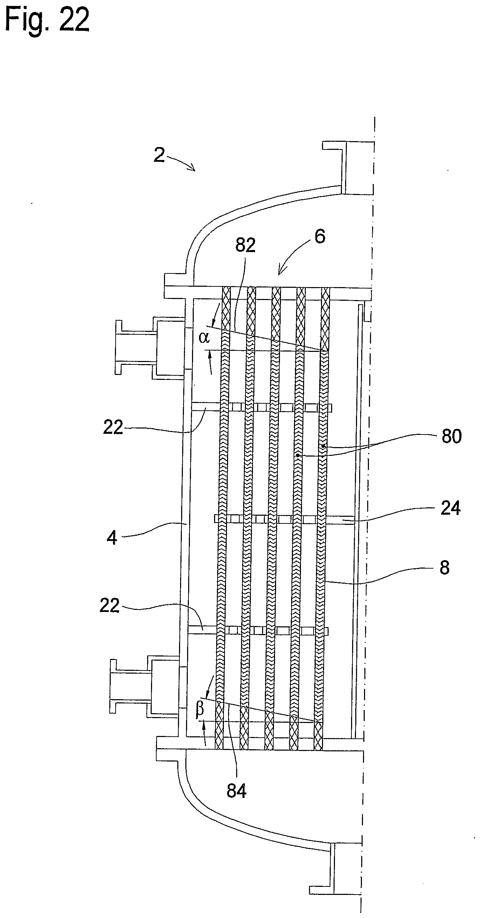

Schließlich kann auch noch die Katalysatorfüllung 80 innerhalb der einzelnen Rohre 8 des Rohrbündels 6 nach Länge, Höhe und/oder katalytischer Aktivität des Katalysators variieren, um so unterschiedlichen Wärmeübergangsbedingungen im Hinblick auf eine hohe Ausbeute und Selektivität Rechnung zu tragen. Fig. 22 zeigt ein Beispiel, wie die Katalysatorfüllungen 80 innerhalb des Rohrbündels 6 ober- wie unterseits durch mit der Mittelachse des Rohrbündels koaxiale Kegel 82 bzw. 84 mit einem Öffnungswinkel $\alpha$ bzw. $\beta$ begrenzt sein können. Zweckmäßigerweise betragen die Öffnungswinkel $\alpha$ und $\beta$ jeweils weniger als 30°, noch zweckmäßiger weniger als 20° und am zweckmäßigsten weniger als 15°. Sie können gleich oder verschieden sein.

Alle vorgenannten Maßnahmen können und werden vielfach zweckmäßigerweise zumindest teilweise nebeneinander Anwendung finden. Ebenso können sie mit Vorteil auch bei besonders gestalteten Mantelrohrreaktoren Anwendung finden, wie etwa einem solchen mit zusätzlichen Ringkanälen und Wärmeträgerkreisläufen und/oder einem Mehrzonenreaktor nach DE-A-2 201 528, einem solchen mit mehreren erst am Aufstellungsort zusammengefügten Sektoren nach DE-A-25 43 758, einem solchen mit mehreren Pumpen nach DE 34 09 159 A1, einem solchen mit wärmeisoliertem oder gekühltem Rohrboden nach DE 198 06 810 A1, einem solchen mit getrennter Zuführung eines zusätzlichen Prozeßgases nach DE 100 21 986 A1, einem solchen mit Nachkühlstufe oder einem solchen mit in ein gemeinsames Reaktorgehäuse integriertem Nachreaktor unter Zwischenschaltung einer Kühlstufe nach DE 101 44 857 A1 (nachveröffentlicht).

Der betreffende Reaktor findet nach derzeitigem Kenntnisstand mit Vorteil Anwendung für Oxidations-, Hydrierungs-, Dehydrierungs-, Nitrierungs-, Alkylierungsprozesse und dergl. mehr und dabei etwa für die Herstellung von Ketonen, Methylisobutylketon, Mercaptan, Isopren, Anthrachinon, o-Kresol, Ethylenhexan, Furfurol, Acetylen, Vinylacetat, Isopropylchlorid, Naphtalsäureanhydrid, Vinylchlorid, Oxoalkohol, Pyrotol, Styrol, Methansäurenitril, Polyphenylenoxid, Dimethylphenol, Pyridinaldehyd, Therban, Alphaolefinen, Vitamin B6, Blausäure, Anilin, Methansäurenitral, Difluormethan, 4-Methyl-2-Pentanon und Tetrahydrofuran sowie im besonderen für die

Oxidation von Dimethylbenzolen (m,o,p) zu den entsprechenden Mono- und Dialdehyden,
Oxidation von Dimethylbenzolen (m,o,p) zu den entsprechenden Mono- und Dicarbonsäuren bzw. deren Anhydriden,
Oxidation von Trimethylbenzolen zu den entsprechenden Mono-, Di- und Trialdehyden,
Oxidation von Trimethylbenzolen zu den entsprechenden Mono-, Di- und Tricarbonsäuren bzw. deren Anhydriden,
Oxidation von Durol zu Pyromellithsäureanhydrid,
Oxidation von gamma- bzw. beta-Picolin zu gamma- bzw. beta-Picolincarbaldehyd,
Oxidation von gamma- bzw. beta-Picolin zu Isonicotinsäure bzw. Nicotinsäure,
Oxidation von Propen zu Acrolein,
Oxidation von Acrolein zu Acrylsäure,
Oxidation von Propan zu Acrolein,
Oxidation von Propan zu Acrylsäure,
Oxidation von Butan zu MSA,
Oxidation von Raffinat zu MSA,

Oxidation von i-Buten zu Methacrolein,

Oxidation von Methacrolein zu Methacrylsäure,

Oxidation von Methacrolein zu Methylmethacrylat,

Oxidation von i-Butan zu Methacrolein,

Oxidation von i-Butan zu Methacrylsäure,

Ammonoxidation von Dimethylbenzolen (m,o,p) zu den entsprechenden Mono- und Dinitrilen,

Ammonoxidation von Trimethylbenzolen zu den entsprechenden Mono- und Di- bzw. Trinitrilen,

Ammonoxidation von Propan zu Acrylnitril,

Ammonoxidation von Propen zu Acrylnitril,

Ammonoxidation von beta-Picolin zu 3-Cyanopyridin,

Ammonoxidation von gamma-Picolin zu 4-Cyanopyridin,

Oxidation von Methanol zu Formaldehyd,

Oxidation von Naphthalin und/oder O-Xylol, ggf. im Mischbetrieb, zu Phthalsäureanhydrid,

Oxidation von Ethan zu Essigsäure,

Oxidation von Ethanol zu Essigsäure,

Oxidation von Geraniol zu Citral,

Oxidation von Ethen zu Ethylenoxid,

Oxidation von Propen zu Propylenoxid,

Oxidation von Chlorwasserstoff zu Chlor,

Oxidation von Glykol zu Glyoxal und

Hydrierung von MSA zu Butandiol.

Es versteht sich, daß, vor allem, wo ein geschmolzenes Salz als Wärmeträger Anwendung findet, zum Anfahren des Reaktors der Wärmeträger dem Wärmeträgerkreislauf bereits in heißem Zustand zugeführt werden oder aber der Wärmeträgerkreislauf einen elektrischen, dampfbeheizten oder befeuerten Erhitzer enthalten muß, um den Wärmeträger auf eine für den Anlauf der Reaktion geeignete Temperatur zu bringen. Solche Maßnahmen sind indessen üblich. Es versteht sich weiter, daß der Reaktor samt Wärmeträgerkreislauf an allen dafür in Betracht kommenden Stellen Entleerungsmittel wie auch mindestens eine Einfüllstelle für den Wärmeträger aufweisen muß.

## Patentansprüche

**1.** Mantelrohrreaktor (2) zur Durchführung katalytischer Gasphasenreaktionen und mit einem von einem im wesentlichen kreiszylindrischen Reaktormantel (4) umgebenen, von einem Wärmeträger umströmten, ringförmigen, vertikalen Reaktionsrohrbündel (6), miteinander abwechselnden ring- und scheibenförmigen Umlenkblechen (22, 24) für den Wärmeträger, die für einen Hindurchtritt des Wärmeträgers Teilstromöffnungen (26) mit zumindest teilweise über den Reaktorradius veränderlichem Querschnitt aufweisen, und Ringkanälen (18, 20) an zumindest beiden Enden des Reaktormantels zur peripheren Zu- bzw. Abführung des Wärmeträgers, die mit einem außenliegenden Wärmetauscher und mindestens einer außenliegenden Umwälzpumpe in Verbindung stehen, wobei die Ringkanäle (18, 20) Fenster (40) aufweisen, die im Reaktormantel (4) ausgebildet sind und durch die hindurch der Wärmeträger dem Inneren des Reaktormantels (4) zugeführt bzw. aus diesem abgeführt wird,
**gekennzeichnet durch**
am Außen- und/oder Innenumfang des Rohrbündels (6) in Umfangsrichtung des Reaktors verteilte Strömungssteuermittel (38, 42, 44) und
in Umfangsrichtung des Reaktors im Sinne kontrollierbarer Rohranströmverhältnisse variierende Querschnitte der Teilstromöffnungen (26) in mindestens einem Kranz in mindestens einer Art der Umlenkbleche (22, 24).

**2.** Mantelrohrreaktor (2) nach Anspruch 1, dadurch **gekenzeichnet,** daß das Verhältnis der durch die Teilstromöffnungen (26) eines jeden Umlenkblechs (22, 24) hindurchtretenden Teilströme zu der gesamten Umwälzmenge des Wärmeträgers zwischen 10 und 80 %, vorzugsweise zwischen 10 und 70 %, noch zweckmäßiger zwischen 10 und 60 % und am zweckmäßigsten zwischen 15 und 30 % beträgt.

**3.** Mantelrohrreaktor (2) nach Anspruch 1 oder 2, **dadurch gekennzeichnet, daß** der zentrale Durchlaß der ringförmigen Umlenkbleche (22) so bemessen ist, daß der dort hindurchtretende Anteil des Wärmeträgers zwischen 10 und 100 %, vorzugsweise zwischen 10 und 85 % und am zweckmäßigsten zwischen 20 und 70 % der Gesamtumwälzmenge des Wärmeträgers beträgt.

**4.** Mantelrohrreaktor (2) nach einem der vorhergehenden Ansprüche, **dadurch gekennzeichnet, daß** der Durchlaß zwischen Außenrand der scheibenförmigen Umlenkbleche (24) und Reaktormantel (4) so bemessen ist, daß der dort hindurchtretende Anteil des Wärmeträgers zwischen 10 und 100 %, vorzugsweise zwischen 15 und 90 % und am zweckmäßigstens zwischen 20 und 80 % der Gesamtumwälzmenge des Wärmeträgers beträgt.

5. Mantelrohrreaktor (2) nach einem der vorhergehenden Ansprüche, **dadurch gekennzeichnet, daß** das Verhältnis zwischen rohrfreier Querschnittsfläche im Inneren des Reaktionsrohrbündels (6) und für die Berohrung verfügbarer Gesamtquerschnittsfläche im Inneren des Reaktormantels 2,25 bis 36 %, vorzugsweise 3 % bis 25 % und am zweckmäßigsten 7 bis 20 % beträgt.

6. Mantelrohrreaktor (2) nach einem der vorhergehenden Ansprüche, **dadurch gekennzeichnet, daß** die Fläche des inneren Durchlasses zumindest eines ringförmigen Umlenkblechs (22) zwischen 30 % und 200 %, vorzugsweise zwischen 40 % und 150 % und am zweckmäßigsten zwischen 70 % und 120 % der Fläche des äußeren Durchlasses mindestens eines scheibenförmigen Umlenkblechs (24) beträgt.

7. Mantelrohrreaktor (2) nach einem der vorhergehenden Ansprüche, **dadurch gekennzeichnet, daß** mindestens eines der Umlenkbleche (22, 24), vorzugsweise der scheibenförmigen Umlenkbleche (24), keine bewußten Teilstromöffnungen (26) aufweist.

8. Mantelrohrreaktor (2) nach einem der vorhergehenden Ansprüche, **dadurch gekennzeichnet, daß** der Abstand von Umlenkblech (22, 24) zu Umlenkblech (24, 22) bzw. von Umlenkblech (22) zu Rohrboden (10, 12) oder - im Falle eines Mehrzonenreaktors - von Umlenkblech (22) zu Trennblech zwischen 30 % und 300 %, vorzugsweise zwischen 50 % und 200 % und am zweckmäßigsten zwischen 70 % und 130 % vom mittleren Abstand beträgt.

9. Mantelrohrreaktor (2) nach einem der vorhergehenden Ansprüche, **dadurch gekennzeichnet, daß** die Teilstromöffnungen (26) der Umlenkbleche (22, 24) zumindest teilweise aus Reaktionsrohre (8) umgebenden Ringspalten bestehen.

10. Mantelrohrreaktor (2) nach einem der vorhergehenden Ansprüche, **dadurch gekennzeichnet, daß** die Teilstromöffnungen (26) zumindest teilweise aus neben bzw. zwischen den Rohrbohrungen (28) in den Umlenkblechen (22, 24) ausgebildeten Durchbrechungen bestehen.

11. Mantelrohrreaktor (2) nach einem der vorhergehenden Ansprüche, **dadurch gekennzeichnet, daß** die Teilstromöffnungen (26) zumindest teilweise aus Erweiterungen (30) der Rohrbohrungen (28) bestehen.

12. Mantelrohrreaktor (2) nach einem der vorhergehenden Ansprüche, **dadurch gekennzeichnet, daß** sich Größe, Dichte und/oder Form der Teilstromöffnungen (26) in radialer Richtung und/oder in Umfangsrichtung des Reaktors ändern.

13. Mantelrohrreaktor (2) nach Anspruch 12, **dadurch gekennzeichnet, daß** Zonen gleicher oder gleich dicht angeordneter Teilstromöffnungen (26) ringförmig und/oder sektorförmig sind.

14. Mantelrohrreaktor (2) nach einem der vorhergehenden Ansprüche, **dadurch gekennzeichnet, daß** zwischen mindestens zwei aufeinanderfolgenden ring- bzw. scheibenförmigen Umlenkblechen (22, 24) bzw. zwischen einem Umlenkblech (22) und einem benachbarten Rohrboden (10, 12) oder Trennblech mindestens ein Strömungsleitblech (48) angeordnet ist.

15. Mantelrohrreaktor (2) nach einem der vorhergehenden Ansprüche, **gekennzeichnet durch** mindestens einen ein oder mehrere Umlenkbleche (22, 24) überbrückenden Bypasskanal (56) für den Wärmeträger.

16. Mantelrohrreaktor (2) nach Anspruch 15, **dadurch gekennzeichnet, daß** die Bypasskanäle (56) innerhalb eines Reaktorquerschnitts so bemessen sind, daß die durch sie hindurchtretenden Kurzschlußströme zwischen 1 und 50 % und vorzugsweise zwischen 10 und 40 % der Gesamtumwälzmenge des Wärmeträgers ausmachen.

17. Mantelrohrreaktor (2) nach einem der vorhergehenden Ansprüche, **dadurch gekennzeichnet, daß** die radial innenseitige und/oder außenseitige Kontur des Rohrbündels (6) einer Wellenlinie folgt.

18. Mantelrohrreaktor (2) nach Anspruch 17, **dadurch gekennzeichnet, daß** die Anordnung der Teilstromöffnungen (26) in den Umlenkblechen entsprechend einer oder mehreren Wellenlinien folgt und/oder der Rand zumindest einzelner der Umlenkbleche (22, 24) und/oder Einbauten, wie Gitter (38), Strömungsleitbleche (48), sonstige Strömungsleiteinrichtungen (42, 44) oder Mischer und/oder die Innen- und/oder Außenkontur zumindest einzelner der Strömungsleitbleche (48) der Kontur des Rohrbündels (6) folgt.

19. Mantelrohrreaktor (2) nach einem der vorhergehenden Ansprüche, **gekennzeichnet durch** Strömungssteuermittel in Form von in ihrer Struktur und/oder ihrem Aufbau über den Reaktorumfang variierenden Einbauten (38; 42; 44), geeignet, dem Wärmeträger ein über den Reaktorumfang variierendes Strömungsprofil zu vermitteln.

20. Mantelrohrreaktor (2) nach einem der vorhergehenden Ansprüche, **gekennzeichnet durch** Einbauten in Form mindestens eines Gitters (38) als turbulenzerzeugendes und/oder Strömungsverteilungsgitter.

21. Mantelrohrreaktor (2) nach einem der vorhergehenden Ansprüche, **gekennzeichnet durch** Strömungssteuermittel in Form mindestens einer Strömungsleiteinrichtung (42; 44) am Übertritt zweier durch ein Umlenkblech (22, 24) voneinander getrennter axialer Reaktorabschnitte (I, II, III, IV).

22. Mantelrohrreaktor (2) nach einem der vorhergehenden Ansprüche, **gekennzeichnet durch** Strömungssteuermittel in Form mindestens eines Mischers am Übertritt zweier durch ein Umlenkblech (22, 24) voneinander getrennter axialer Reaktorabschnitte (I, II, III, IV) und/oder am Wärmeträgereintritt in einen solchen.

23. Mantelrohrreaktor (2) nach einem der vorhergehenden Ansprüche, **gekennzeichnet durch** Strömungssteuermittel in Form von Leitflächen in und/oder an den den wärmeträgereintrittsseitigen Ringkanal (20) mit dem Inneren des Reaktormantels (4) verbindenden Fenstern (40).

24. Mantelrohrreaktor (2) nach einem der vorhergehenden Ansprüche, **dadurch gekennzeichnet, daß** der Wärmeübergangswert an den Kontaktrohren (8) auf mindestens 1000, vorzugsweise mindestens 1500, noch zweckmäßiger mindestens 2000 und am zweckmäßigsten mindestens 2500 Wm$^2$°K eingestellt ist.

25. Mantelrohrreaktor (2) nach Anspruch 1, **gekennzeichnet durch** über den Reaktorumfang reichende oder verteilte turbulenzfördernde Strömungssteuermittel (38, 42, 44) in Gestalt eines Gitters (38) am Außen- und/oder Innenumfang des Rohrbündels (6).

26. Mantelrohrreaktor (2) nach Anspruch 1, **gekennzeichnet durch** eine zumindest im wesentlichen in Umfangrichtung des Reaktors im Sinne kontrollierbarer Strömungswiderstände variierende Rohrteilung des Rohrbündels (6).

27. Mantelrohrreaktor (2) nach Anspruch 1, **gekennzeichnet durch** eine in Umfangsrichtung des Reaktors im Sinne kontrollierbarer Strömungswiderstände variierende radiale Dimension des Rohrbündels (6) und/oder mindestens einer Art der Umlenkbleche (22, 24).

28. Mantelrohrreaktor (2) nach Anspruch 1, **dadurch gekennzeichnet, daß** sämtliche Rohre (8) des Reaktionsrohrbündels (6) in zumindest einem der Umlenkbleche (22, 24) abgestützt sind.

29. Verwendung eines Mantelrohrreaktors (2) nach einem der vorhergehenden Ansprüche für Oxidations-, Hydrierungs-, Dehydrierungs-, Nitrierungs-, Alkylierungsprozese.

30. Verwendung nach Anspruch 29 für die Herstellung von Ketonen, Methylisobutylketon, Mercaptan, Isopren, Anthrachinon, o-Kresol, Ethylenhexan, Furfurol, Acetylen, Vinylacetat, Isopropylchlorid, Naphtalsäureanhydrid, Vinylchlorid, Oxoalkohol, Pyrotol, Styrol, Methansäurenitril, Polyphenylenoxid, Dimethylphenol, Pyridinaldehyd, Therban, Alphaolefinen, Vitamin B6, Blausäure, Anilin, Methansäurenitral, Difluormethan, 4-Methyl-2-Pentanon und Tetrahydrofuran sowie im besonderen die
Oxidation von Dimethylbenzolen (m,o,p) zu den entsprechenden Mono- und Dialdehyden,
Oxidation von Dimethylbenzolen (m,o,p) zu den entsprechenden Mono- und Dicarbonsäuren bzw. deren Anhydriden,
Oxidation von Trimethylbenzolen zu den entsprechenden Mono-, Di- und Trialdehyden,
Oxidation von Trimethylbenzolen zu den entsprechenden Mono-, Di- und Tricarbonsäuren bzw. deren Anhydriden,
Oxidation von Durol zu Pyromellithsäureanhydrid,
Oxidation von gamma- bzw. beta-Picolin zu gamma- bzw. beta-Picolincarbaldehyd,
Oxidation von gamma- bzw. beta-Picolin zu Isonicotinsäure bzw. Nicotinsäure,
Oxidation von Propen zu Acrolein,
Oxidation von Acrolein zu Acrylsäure,
Oxidation von Propan zu Acrolein,

Oxidation von Propan zu Acrylsäure,
Oxidation von Butan zu MSA,
Oxidation von Raffinat zu MSA,
Oxidation von i-Buten zu Methacrolein,
Oxidation von Methacrolein zu Methacrylsäure,
Oxidation von Methacrolein zu Methylmethacrylat,
Oxidation von i-Butan zu Methacrolein,
Oxidation von i-Butan zu Methacrylsäure,
Ammonoxidation von Dimethylbenzolen (m,o,p) zu den entsprechenden Mono- und Dinitrilen,
Ammonoxidation von Trimethylbenzolen zu den entsprechenden Mono- und Di- bzw. Trinitrilen,
Ammonoxidation von Propan zu Acrylnitril,
Ammonoxidation von Propen zu Acrylnitril,
Ammonoxidation von beta-Picolin zu 3-Cyanopyridin,
Ammonoxidation von gamma-Picolin zu 4-Cyanopyridin, Oxidation von Methanol zu Formaldehyd,
Oxidation von Naphthalin und/oder O-Xylol, ggf. im Mischbetrieb, zu Phthalsäureanhydrid,
Oxidation von Ethan zu Essigsäure,
Oxidation von Ethanol zu Essigsäure,
Oxidation von Geraniol zu Citral,
Oxidation von Ethen zu Ethylenoxid,
Oxidation von Propen zu Propylenoxid,
Oxidation von Chlorwasserstoff zu Chlor,
Oxidation von Glykol zu Glyoxal und
Hydrierung von MSA zu Butandiol.

## Claims

1. A shell-and-tube type reactor (2) for carrying out catalytic gas phase reactions and comprising a ring-shaped vertical reaction tube bundle (6) surrounded by a substantially circular cylindrical reactor shell (4) and having a heat carrier flowing therethrough; alternating ring-shaped and disc-shaped baffle plates (22, 24) for the heat carrier, which comprise partial flow openings (26), having a cross-section that is at least partially variable over the reactor radius, for the heat carrier to pass; and ring channels (18, 20) provided at least at both ends of the reactor shell for peripherally supplying or discharging the heat carrier, which are in communication with an external heat exchanger and at least one external circulating pump, wherein the ring channels (18, 20) comprise openings (40), which are formed in the reactor shell (4) and through which the heat carrier is supplied to the interior of the reactor shell (4) or discharged therefrom,
**characterized by**
flow control means (38, 42, 44) distributed at the outer and/or inner circumference of the tube bundle (6) in the circumferential direction of the reactor, and
in that
the partial flow openings (26) within at least one ring within at least one type of the baffle plates (22, 24) have cross-sections that vary in the circumferential direction of the reactor in terms of controllable conditions for a flow against the tubes.

2. The shell-and-tube type reactor (2) according to claim 1, **characterized in that** the ratio between the partial flows passing through the partial flow openings (26) of each baffle plate (22, 24) and the total circulation amount of the heat carrier is between 10 and 80%, preferably between 10 and 70%, even more advantageously between 10 and 60% and most advantageously between 15 and 30%.

3. The shell-and-tube type reactor (2) according to claim 1 or 2, **characterized in that** the central passage of the ring-shaped baffle plates (22) is dimensioned such that the proportion of the heat carrier passing therethrough is between 10 and 100 %, preferably between 10 and 85 % and most advantageously between 20 and 70 % of the total circulation amount of the heat carrier.

4. The shell-and-tube type reactor (2) according to any of the preceding claims, **characterized in that** the passage between the outer edge of the disc-shaped baffle plates (24) and the reactor shell (4) is dimensioned such that the proportion of the heat carrier passing therethrough is between 10 and 100 %, preferably between 15 and 90 %, and most advantageously between 20 and 80 % of the total circulation amount of the heat carrier.

**5.** The shell-and-tube type reactor (2) according to any of the preceding claims, **characterized in that** the ratio between the tube-free cross-sectional area in the interior of the reaction tube bundle (6) and the total cross-sectional area in the interior of the reactor shell available for the tubing is 2.25 to 36 %, preferably 3 to 25 % and most advantageously 7 to 20 %.

**6.** The shell-and-tube type reactor (2) according to any of the preceding claims, **characterized in that** the area of the internal passage of at least one ring-shaped baffle plate (22) ranges between 30 % and 200 %, preferably between 40 % and 150 %, and most advantageously between 70 % and 120 % of the area of the outer passage of at least one disc-shaped baffle plate (24).

**7.** The shell-and-tube type reactor (2) according to any of the preceding claims, **characterized in that** at least one of the baffle plates (22, 24), preferably of the disc-shaped baffle plates (24), has no intentional partial flow openings (26).

**8.** The shell-and-tube type reactor (2) according to any of the preceding claims, **characterized in that** the distance between the baffle plate (22, 24) and the baffle plate (24, 22), or between the baffle plate (22) and the tube sheet (10, 12), respectively, or - in the case of a multi-zone reactor - between the baffle plate (22) and the partition plate, ranges between 30 % and 300 %, preferably between 50 % and 200 % and most advantageously between 70 % and 130 % of the mean distance.

**9.** The shell-and-tube type reactor (2) according to any of the preceding claims, **characterized in that** the partial flow openings (26) of the baffle plates (22, 24) consist at least partially of annular gaps surrounding the reaction tubes (8).

**10.** The shell-and-tube type reactor (2) according to any of the preceding claims, **characterized in that** the partial flow openings (26) consist at least partially of apertures formed next to or between the tube holes (28) within the baffle plates (22, 24).

**11.** The shell-and-tube type reactor (2) according to any of the preceding claims, **characterized in that** the partial flow openings (26) consist at least partially of extensions (30) of the tube holes (28).

**12.** The shell-and-tube type reactor (2) according to any of the preceding claims, **characterized in that** the size, density and/or shape of the partial flow openings (26) change in the radial direction and/or in the circumferential direction of the reactor.

**13.** The shell-and-tube type reactor (2) according to claim 12, **characterized in that** zones of equal partial flow openings (26), or partial flow openings (26) arranged with equal density, are annular and/or sector-shaped.

**14.** The shell-and-tube type reactor (2) according to any of the preceding claims, **characterized in that** at least one flow guiding plate (48) is arranged between at least two successive ring-shaped or disc-shaped baffle plates (22, 24), respectively, or between a baffle plate (22) and an adjacent tube sheet (10, 12) or partition plate, respectively.

**15.** The shell-and-tube type reactor (2) according to any of the preceding claims, **characterized by** at least one bypass channel (56), bridging one or more baffle plates (22, 24), for the heat carrier.

**16.** The shell-and-tube type reactor (2) according to claim 15, **characterized in that** the bypass channels (56) are, within a reactor cross-section, dimensioned such that the short-circuit flows passing therethrough account for between 1 and 50%, and preferably between 10 and 40% of the total circulation amount of the heat carrier.

**17.** The shell-and-tube type reactor (2) according to any of the preceding claims, **characterized in that** the radially inside and/or radially outside contour of the tube bundle (6) follows a wavy line.

**18.** The shell-and-tube type reactor (2) according to claim 17, **characterized in that** the arrangement of the partial flow openings (26) within the baffle plates accordingly follows one or more wavy lines and/or the edge of at least some of the baffle plates (22, 24) and/or internals, such as grids (38), flow guiding plates (48), other flow guiding means (42, 44) or mixers, and/or the inner and/or outer contour of at least some of the flow guiding plates (48) follows the contour of the tube bundle (6).

**19.** The shell-and-tube type reactor (2) according to any of the preceding claims, **characterized by** flow control means in the form of internals (38; 42; 44), which vary in their structure and/or their design over the reactor circumference and are suitable for imparting a flow profile, which varies over the reactor circumference, to the heat carrier.

20. The shell-and-tube type reactor (2) according to any of the preceding claims, **characterized by** internals in the form of at least one grid (38) serving as a turbulence-generating and/or flow-distributing grid.

21. The shell-and-tube type reactor (2) according to any of the preceding claims, **characterized by** flow control means in the form of at least one flow guiding means (42; 44) at the junction of two axial reactor sections (I, II, III, IV) separated from one another by a baffle plate (22, 24).

22. The shell-and-tube type reactor (2) according to any of the preceding claims, **characterized by** flow control means in the form of at least one mixer at the junction of two axial reactor sections (I, II, III, IV) separated from one another by a baffle plate (22, 24), and/or at the point where the heat carrier enters into such reactor section.

23. The shell-and-tube type reactor (2) according to any of the preceding claims, **characterized by** flow control means in the form of guiding faces within and/or at the openings (40) connecting the ring-shaped channel (20) on the heat carrier entry side to the interior of the reactor shell (4).

24. The shell-and-tube type reactor (2) according to any of the preceding claims, **characterized in that** the heat transfer value at the contact tubes (8) is set to at least 1000, preferably at least 1500, even more advantageously at least 2000 and most advantageously at least 2500 W/m$^2$°K.

25. The shell-and-tube type reactor (2) according to claim 1, **characterized by** turbulence-promoting flow control means (38, 42, 44), extending or being distributed over the reactor circumference, in the form of a grid (38) provided at the outer and/or inner circumference of the tube bundle (6).

26. The shell-and-tube type reactor (2) according to claim 1, **characterized by** a tube pitch of the tube bundle (6) varying, at least substantially, in the circumferential direction of the reactor in terms of controllable flow resistances.

27. The shell-and-tube type reactor (2) according to Claim 1, **characterized by** a radial dimension of the tube bundle (6) and/or of at least one type of the baffle plates (22, 24) varying in the circumferential direction of the reactor in terms of controllable flow resistances.

28. The shell-and-tube type reactor (2) according to claim 1, **characterized in that** all tubes (8) of the reaction tube bundle (6) are supported in at least one of the baffle plates (22, 24).

29. Use of a shell-and-tube type reactor (2) according to any of the preceding claims for oxidation, hydrogenation, dehydrogenation, nitration, alkylation processes.

30. The use according to claim 29 for the production of ketones, methyl isobutyl ketone, mercaptan, isoprene, anthraquinone, o-cresol, ethylene hexane, furfurol, acetylene, vinyl acetate, isopropyl chloride, naphthalic anhydride, vinyl chloride, oxo alcohol, pyrotol, styrene, methanoic nitrile, polyphenylene oxide, dimethyl phenol, pyridine aldehyde, therban, alpha-olefins, vitamin B6, hydrocyanic acid, aniline, methanoic nitral, difluoromethane, 4-methyl-2-pentanone and tetrahydrofuran and in particular the
oxidation of dimethylbenzenes (m,o,p) to the corresponding mono- and dialdehydes,
oxidation of dimethylbenzenes (m,o,p) to the corresponding mono- and dicarboxylic acids or their anhydrides,
oxidation of trimethylbenzenes to the corresponding mono-, di- and trialdehydes,
oxidation of trimethylbenzenes to the corresponding mono-, di- and tricarboxylic acids or their anhydrides,
oxidation of durene to pyromellitic anhydride,
oxidation of gamma- or beta-picoline to gamma- or beta-picoline carbaldehyde,
oxidation of gamma- or beta-picoline to isonicotinic acid or nicotinic acid,
oxidation of propene to acrolein,
oxidation of acrolein to acrylic acid,
oxidation of propane to acrolein,
oxidation of propane to acrylic acid,
oxidation of butane to MSA,
oxidation of raffinate to MSA,
oxidation of i-butene to methacrolein,
oxidation of methacrolein to methacrylic acid,

oxidation of methacrolein to methyl methacrylate,

oxidation of i-butane to methacrolein,

oxidation of i-butane to methacrylic acid,

ammonoxidation of dimethylbenzenes (m,o,p) to the corresponding mono- and dinitriles,

ammonoxidation of trimethylbenzenes to the corresponding mono- and di- or trinitriles,

ammonoxidation of propane to acrylonitrile,

ammonoxidation of propene to acrylonitrile,

ammonoxidation of beta-picoline to 3-cyanopyridine,

ammonoxidation of gamma-picoline to 4-cyanopyridine,

oxidation of methanol to formaldehyde,

oxidation of naphthalene and/or o-xylene, if required in a mixing operation, to phthalic anhydride,

oxidation of ethane to acetic acid,

oxidation of ethanol to acetic acid,

oxidation of geraniol to citral,

oxidation of ethene to ethylene oxide,

oxidation of propene to propylene oxide,

oxidation of hydrogen chloride to chlorine,

oxidation of glycol to glyoxal and

hydrogenation of MSA to butanediol.

**Revendications**

1. Réacteur tubulaire à enveloppe (2) pour l'exécution de réactions catalytiques en phase gazeuse et comprenant un faisceau de tubes de réaction (6) annulaire, vertical, immergé dans un fluide caloporteur et entouré par une enveloppe de réacteur (4) sensiblement cylindrique circulaire, des tôles de déflexion (22, 24) alternantes et en forme d'anneau et de disque pour le fluide caloporteur qui, pour un passage du fluide caloporteur, présentent des ouvertures d'écoulement partiel (26) ayant une section transversale au moins partiellement variable sur le rayon du réacteur, et des canaux annulaires (18, 20) au moins aux deux extrémités de l'enveloppe de réacteur pour l'alimentation ou l'évacuation périphérique du fluide caloporteur, qui sont en communication avec un échangeur de chaleur externe et avec au moins une pompe de recirculation externe, les canaux annulaires (18, 20) présentant des fenêtres (40) qui sont ménagées dans l'enveloppe de réacteur (4) et à travers lesquelles le fluide caloporteur est alimenté vers l'intérieur de l'enveloppe de réacteur (4) ou évacué hors de celle-ci,
   **caractérisé par**
   des moyens de commande d'écoulement (38, 42, 44) répartis sur la périphérie extérieure et/ou intérieure du faisceau de tubes (6) en direction périphérique du réacteur, et par
   des ouvertures d'écoulement partiel (26) à sections transversales qui varient en direction périphérique du réacteur dans le sens de rapports d'attaque de tube contrôlables, dans au moins une couronne dans au moins un type des tôles de déflexion (22, 24).

2. Réacteur tubulaire à enveloppe (2) selon la revendication 1, **caractérisé en ce que** le rapport des courants partiels traversant les ouvertures d'écoulement partiel (26) de chaque tôle de déflexion (22, 24) sur la totalité de la quantité recirculée du fluide caloporteur est entre 10 et 80 %, de préférence entre 10 et 70 %, judicieusement entre 10 et 60 % et encore mieux entre 15 et 30 %.

3. Réacteur tubulaire à enveloppe (2) selon la revendication 1 ou 2, **caractérisé en ce que** le passage central des tôles de déflexion annulaires (22) est dimensionné de telle sorte que la part du fluide caloporteur qui passe ici est entre 10 et 100 %, de préférence entre 10 et 85 % et judicieusement entre 20 et 70 % de la quantité recirculée totale du fluide caloporteur.

4. Réacteur tubulaire à enveloppe (2) selon l'une des revendications précédentes, **caractérisé en ce que** le passage entre le bord extérieur des tôles de déflexion (24) en forme de disques et l'enveloppe de réacteur (4) est dimensionné de telle sorte que la part du fluide caloporteur qui passe ici est entre 10 et 100 %, de préférence entre 15 et 90 % et judicieusement entre 20 et 80 % de la quantité recirculée totale du fluide caloporteur.

5. Réacteur tubulaire à enveloppe (2) selon l'une des revendications précédentes, **caractérisé en ce que** le rapport de la surface de section transversale dépourvue de tube à l'intérieur du faisceau de tubes de réaction (6) sur la surface totale de la section transversale disponible pour la tuyauterie à l'intérieur de l'enveloppe de réacteur est de 2,25 à 36 %, de préférence de 3 % à 25 % et judicieusement de 7 à 20 %.

6. Réacteur tubulaire à enveloppe (2) selon l'une des revendications précédentes, **caractérisé en ce que** la surface du passage intérieur d'au moins une tôle de déflexion annulaire (22) est entre 30 et 200 %, de préférence entre 40 % et 150 % et judicieusement entre 70 % et 120 % de la surface du passage extérieur d'au moins une tôle de déflexion (24) en forme de disque.

7. Réacteur tubulaire à enveloppe (2) selon l'une des revendications précédentes, **caractérisé en ce que** l'une au moins des tôles de déflexion (22, 24), de préférence des tôles de déflexion (24) en forme de disque ne présente pas d'ouvertures d'écoulement partiel (26) intentionnelles.

8. Réacteur tubulaire à enveloppe (2) selon l'une des revendications précédentes, **caractérisé en ce que** la distance d'une tôle de déflexion (22, 24) à une autre tôle de déflexion (24, 22) ou d'une tôle de déflexion (22) au fond de tube (10, 12) ou - dans le cas d'un réacteur multizone - d'une tôle de déflexion (22) à la tôle de séparation est entre 30 % et 300 %, de préférence entre 50 % et 200 % et judicieusement entre 70 % et 130 % de la distance moyenne.

9. Réacteur tubulaire à enveloppe (2) selon l'une des revendications précédentes, **caractérisé en ce que** les ouvertures d'écoulement partiel (26) des tôles de déflexion (22, 24) sont constituées au moins partiellement par des fentes annulaires entourant les tubes de réaction (8).

10. Réacteur tubulaire à enveloppe (2) selon l'une des revendications précédentes, **caractérisé en ce que** les ouvertures d'écoulement partiel (26) sont constituées au moins partiellement par des traversées ménagées à côté ou entre les perçages pour tube (28) dans les tôles de déflexion (22, 24).

11. Réacteur tubulaire à enveloppe (2) selon l'une des revendications précédentes, **caractérisé en ce que** les ouvertures d'écoulement partiel (26) sont constituées au moins partiellement par des évasements (30) des perçages pour tube (28).

12. Réacteur tubulaire à enveloppe (2) selon l'une des revendications précédentes, **caractérisé en ce que** la taille, la densité et/ou la forme des ouvertures d'écoulement partiel (26) se modifient en direction radiale et/ou en direction périphérique du réacteur.

13. Réacteur tubulaire à enveloppe (2) selon la revendication 12, **caractérisé en ce que** des zones des mêmes ouvertures d'écoulement partiel (26) ou des ouvertures agencées avec la même densité sont annulaires et/ou en forme de secteur.

14. Réacteur tubulaire à enveloppe (2) selon l'une des revendications précédentes, **caractérisé en ce qu'**au moins une tôle de guidage d'écoulement (48) est agencée entre au moins deux tôles de déflexion (22, 24) annulaires ou en forme de disque successives ou entre une tôle de déflexion (22) et un fond de tube voisin (10, 12) ou une tôle de séparation.

15. Réacteur tubulaire à enveloppe (2) selon l'une des revendications précédentes, **caractérisé par** au moins un canal formant by-pass (56) pour le fluide caloporteur, qui ponte une ou plusieurs tôles de déflexion (22, 24).

16. Réacteur tubulaire à enveloppe (2) selon la revendication 15, **caractérisé en ce que** les canaux formant by-pass (56) à l'intérieur d'une section transversale de réacteur sont dimensionnés de telle sorte que les courants de court-circuit qui les traversent font entre 1 et 50 % et de préférence entre 10 et 40 % de la quantité recirculée totale du fluide caloporteur.

17. Réacteur tubulaire à enveloppe (2) selon l'une des revendications précédentes, **caractérisé en ce que** le contour radialement intérieur et/ou extérieur du faisceau de tubes (6) suit une ligne ondulée.

18. Réacteur tubulaire à enveloppe (2) selon la revendication 17, **caractérisé en ce que** la disposition des ouvertures d'écoulement partiel (26) dans les tôles de déflexion suit en correspondance une ou plusieurs lignes ondulées et/ou le bord de quelques-unes au moins des tôles de déflexion (22, 24) et/ou des inserts, tels que des grilles (38), des tôles de guidage d'écoulement (48), d'autres moyens de guidage d'écoulement (42, 44) ou des mélangeurs, et/ou le contour intérieur et/ou extérieur de quelques-unes au moins des tôles de guidage d'écoulement (48) suit le contour du faisceau de tubes (6).

19. Réacteur tubulaire à enveloppe (2) selon l'une des revendications précédentes, **caractérisé par** des moyens de commande d'écoulement sous la forme d'inserts (38 ; 42 ; 44) qui varient quant à leur structure et/ou à leur construction sur la périphérie du réacteur, qui sont aptes à imposer au fluide caloporteur un profil d'écoulement qui varie sur la périphérie du réacteur.

20. Réacteur tubulaire à enveloppe (2) selon l'une des revendications précédentes, **caractérisé par** des inserts sous la forme d'une grille (38) à titre de grille de génération de turbulences et/ou de répartition d'écoulement.

21. Réacteur tubulaire à enveloppe (2) selon l'une des revendications précédentes, **caractérisé par** des moyens de commande d'écoulement sous la forme d'au moins un moyen de guidage d'écoulement (42 ; 44) à la transition de deux sections de réacteur (I, II, III, IV) axiales séparées l'une de l'autre par une tôle de déflexion (22, 24).

22. Réacteur tubulaire à enveloppe (2) selon l'une des revendications précédentes, **caractérisé par** des moyens de commande d'écoulement sous la forme d'au moins un mélangeur à la transition de deux sections de réacteur (I, II, III, IV) axiales séparées l'une de l'autre par une tôle de déflexion (22, 24) et/ou à l'entrée du fluide caloporteur dans une telle section.

23. Réacteur tubulaire à enveloppe (2) selon l'une des revendications précédentes, **caractérisé par** des moyens de commande d'écoulement sous la forme de surfaces de guidage et/ou dans et/ou sur des fenêtres (40) reliant le canal annulaire (20) côté entrée de fluide caloporteur au volume intérieur de l'enveloppe de réacteur (4).

24. Réacteur tubulaire à enveloppe (2) selon l'une des revendications précédentes, **caractérisé en ce que** le coefficient de transfert thermique au niveau des tubes de contact (8) est réglé à au moins 1000, de préférence à au moins 1500, judicieusement à au moins 2000 et le mieux à au moins 2500 $W/m^{2\circ}K$.

25. Réacteur tubulaire à enveloppe (2) selon la revendication 1, **caractérisé par** des moyens de commande d'écoulement (38, 42, 44) favorisant les turbulences et réalisés sous la forme d'une grille (38) à la périphérie extérieure et/ou intérieure du faisceau de tubes (6), qui s'étendent ou sont répartis sur la périphérie du réacteur.

26. Réacteur tubulaire à enveloppe (2) selon la revendication 1, **caractérisé par** une répartition du faisceau de tubes (6) qui varie au moins sensiblement en direction périphérique du réacteur dans le sens de résistances à l'écoulement contrôlables.

27. Réacteur tubulaire à enveloppe (2) selon la revendication 1, **caractérisé par** une dimension radiale du faisceau de tubes (6) et/ou d'au moins un type de tôles de déflexion (22, 24), qui varie en direction périphérique du réacteur dans le sens de résistances à l'écoulement contrôlables.

28. Réacteur tubulaire à enveloppe (2) selon la revendication 1, **caractérisé en ce que** tous les tubes (8) du faisceau de tubes de réaction (6) sont soutenus dans l'une au moins des tôles de déflexion (22, 24).

29. Utilisation d'un réacteur tubulaire à enveloppe (2) selon l'une des revendications précédentes pour des processus d'oxydation, d'hydratation, de déshydratation, de nitruration, d'alkylation.

30. Utilisation selon la revendication 29 pour la production de cétones, méthylisobutylcétone, mercaptan, isoprène, anthraquinone, o-crésol, éthylène hexane, furfurol, acétylène, acétate de vinyle, chlorure d'isopropyle, anhydride naphtalique, chlorure de vinyle, oxoalcool, pyrotole, styrène, méthanesonitrile, polyphénylène oxyde, diméthylphénol, pyridine aldéhyde, therban, alphaoléfines, vitamine B6, acide cyanhydrique, aniline, nitrure d'acide formique, difluorométhane, 4-méthyl-2-pentanone et tétrahydrofurane et en particulier
oxydation de diméthylbenzènes (m, o, p) en mono- et dialdéhydes correspondants,
oxydation de diméthylbenzènes (m, o, p) en acides mono- et dicarboxyliques correspondants ou leurs anhydrides,
oxydation de triméthylbenzènes en mono-, di- et trialdéhydes correspondants,
oxydation de triméthylbenzènes en acides mono-, di- et tricarboxyliques correspondants ou leurs anhydrides,
oxydation de durol en anhydride d'acide pyromellitique,
oxydation de gamma- ou de bêta-picoline en gamma- ou bêta-picoline carbaldéhyde,
oxydation de gamma- ou de bêta-picoline en acide isonicotinique ou en acide nicotinique,
oxydation de propène en acroléine,
oxydation d'acroléine en acide acrylique,
oxydation de propane en acroléine,

oxydation de propane en acide acrylique,

oxydation de butane en MSA,

oxydation de raffinat en MSA,

oxydation de i-butène en méthacroléine,

oxydation de méthacroléine en acide méthacrylique,

oxydation de méthacroléine en méthacrylate de méthyle,

oxydation de i-butane en méthacroléine,

oxydation de i-butane en acide méthacrylique,

ammoxydation de diméthylbenzènes (m, o, p) en mono- et dinitriles correspondants,

ammoxydation de triméthylbenzènes en mono- et di- ou trinitriles correspondants,

ammoxydation de propane en acrylonitrile,

ammoxydation de propène en acrylonitrile,

ammoxydation de beta-picoline en 3-cyanopyridine,

ammoxydation de gamma-picoline en 4-cyanopyridine,

oxydation de méthanol en formaldéhyde,

oxydation de naphtalène et/ou d'o-xylène, si nécessaire en mélange, en anhydride d'acide phtalique,

oxydation d'éthane en acide acétique,

oxydation d'éthanol en acide acétique,

oxydation de géraniol en citral,

oxydation d'éthène en oxyde d'éthylène,

oxydation de propène en oxyde de propylène,

oxydation du chlorure d'hydrogène en chlore,

oxydation de glycol en glyoxal et

hydrogénation de MSA en butanediol.

## Fig. 1

# Fig. 2

# Fig. 3

# Fig. 4

a) 22, 24  26  28
b) 26  28
c) 26  28
d) 28  30 (26)
e) 30 (26)  28  30 (26)
f) 30 (26)  28  30 (26)

# Fig. 5

a)

32

8

26

22, 24

b)

8

26

32

8

22, 24

# Fig. 6

a)

8

22, 24

28

b)

8

22, 24

34

# Fig. 7

# Fig. 8

a)

0°

6

4

b)

0°

6

4

## Fig. 9

a)

8

b)

8

# Fig. 10

I    II

Fig. 11

## Fig. 12

# Fig. 13

## Fig. 14

## Fig. 15

# Fig. 16

Fig. 17

# Fig. 18

a)

50

4    40

b)

50

4    40

c)

50    50

4    40

Fig. 19

# Fig. 20

## Fig. 21

EP 1 569 745 B1

# Fig. 22

40

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 2201528 A
- DE 4431957 A
- DE 1601162 A
- DE 1675501 A
- GB 310157 A
- EP 0382098 B1
- EP 1080780 A
- DE 1675501 C3
- DE 2559661 A
- DE 2750824 A

- EP 0382098 A
- US 4357991 A
- DE 19909340 A1
- WO 9006807 A
- DE 10024342 A1
- DE 2543758 A
- DE 3409159 A1
- DE 19806810 A1
- DE 10021986 A1
- DE 10144857 A1